(19)

**Europäisches Patentamt**
**European Patent Office**
**Office européen des brevets**

(11) **EP 2 073 004 A1**

(12) **EUROPEAN PATENT APPLICATION**

(43) Date of publication:
**24.06.2009 Bulletin 2009/26**

(51) Int Cl.:
***G01N 33/50*** (2006.01)

(21) Application number: **07024623.6**

(22) Date of filing: **19.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Helmholtz Zentrum München Deutsches Forschungszentrum für Gesundheit und Umwelt (GmbH) 85764 Neuherberg (DE)**

(72) Inventor: **Zischka, Hans 80469 München (DE)**

(74) Representative: **Vossius & Partner Siebertstrasse 4 81675 München (DE)**

(54) **Electrophoretic analysis of isolated mitochondria for the detection of cell or tissue damage**

(57) A method for detecting mitochondrial damage comprising: (a) isolating mitochondria from a sample; (b1) separating populations by means of their electrophoretic mobility; and (c1) comparing the populations with a reference sample, wherein a populations rise, a population(s) shift towards the anode or the cathode and/or an increase in population heterogeneity is indicative of mitochondrial damage; or (b2) analyzing the average surface charge of the isolated mitochondria; and (c2) comparing the average surface charge with a reference sample wherein a shift of the average surface charge as compared to that of the reference sample is indicative of mitochondrial damage; and

(d) confirming that mitochondrial damage is present. Furthermore, the present invention relates to a method of identifying an agent causing mitochondrial damage in mammalian cells and a method of identifying an agent capable of improving the condition of mammalian mitochondria.

**Description**

The present invention relates to a method for detecting mitochondrial damage in mammalian tissue or cells comprising: (a) isolating mitochondria from a sample obtained from a mammal and containing tissue or cells containing mitochondria; (b1) separating populations of the isolated mitochondria of step (a) by means of their electrophoretic mobility; and (c1) comparing the populations of the mitochondria obtained in step (b1) with those of a reference sample containing tissue or cells displaying a non-pathological phenotype, wherein a rise in the number of populations, a shift of the population(s) towards the anode or the cathode and/or an increase in the heterogeneity of one or more populations separated in step (b1) as compared to those of the reference sample is indicative of mitochondrial damage; or (b2) analyzing the average surface charge of the isolated mitochondria of step (a); and (c2) comparing the average surface charge determined in step (b2) with that of a reference sample containing cells displaying a non-pathological phenotype, wherein a shift of the average surface charge of the mitochondria analyzed in step (b2) as compared to that of the mitochondria of the reference sample is indicative of mitochondrial damage; and (d) in the case that a shift of the population(s) towards the cathode in step (c1) or a shift of the average surface charge to the positive in step (c2) is detected or, confirming that mitochondrial damage is present. Furthermore, the present invention relates to a method of identifying an agent causing mitochondrial damage in mammalian cells and a method of identifying an agent capable of improving the condition of mammalian mitochondria.

In this specification, a number of documents are cited. The disclosure content of these documents including manufacturers' manuals is herewith incorporated by reference in its entirety.

Mitochondria are the powerhouse of the cell and linked to many fundamental cellular processes. They are involved in many metabolic and anabolic processes and play an important role in cell death in higher eukaryotic life. Low mitochondrial production of reactive oxygen species (ROS) has been linked to longevity of the cell.

Cell fate in terms of apoptosis or/and necrosis is frequently determined by one crucial event: mitochondrial outer membrane permeabilization (MOMP). MOMP marks the "point of no return" for the cell, after which it is committed to death. MOMP leads to the release of proteins normally found in the space between the inner and outer mitochondrial membranes (e.g. cytochrome c and AIF). MOMP may be induced directly or may be the culmination of a sequence of events starting with an increase in the permeability of the mitochondrial inner membrane called permeability transition (PT). PT was shown to be unspecifically induced e.g. by calcium (Hunter at al., 1976). Since then a plethora of studies have dealt with this phenomenon which investigated the mechanistic, molecular and pharmacological aspects of PT. It was shown that PT could be inhibited by submicromolar concentrations of the immunosuppressant drug cyclosporine A and that it involves the creation of a pore/channel-like structure (Crompton et al., 1988; Sorgato et al., 1987; Szabo and Zoratti, 1992). As had been realized early on, the increased permeability of solutes towards the matrix space causes an osmotically driven water influx. The concomitant extension of the mitochondrial inner membrane ("swelling") leads to a matrix transition from the "aggregated" to the "orthodox" state and finally results in MOMP (Hunter et al., 1976; Petronilli et al., 1993; Van der Heiden et al., 1997; Brenner and Grimm, 2006).

The molecular constituents of the PT pore have remained elusive (Bernardi et al., 2006; Grimm and Brdiczka, 2007; Saris and Carafoli, 2005; Baines et al., 2007). In addition, although it is generally accepted that the PT is an "all-or-nothing" event (Petronilli et al., 1993), it has been proposed that subpopulations of mitochondria differ in their thresholds to undergo PT (Petronilli et al., 1993; Beatrice et al., 1992). Unfortunately, there is currently no method at hand to enrich and subsequently characterize these subpopulations.

Moreover, a large number of distinct stimuli can trigger PT (Green and Kroemer, 2004). Various factors enhance the likelihood of PT pore opening. In some mitochondria, such as those in the central nervous system, high levels of $Ca^{2+}$ within mitochondria can cause the PT pore to open (Brustovetsky et al., 2003; Hunter et al., 1976). This is possibly because $Ca^{2+}$ binds to and activates $Ca^{2+}$ binding sites on the matrix side of the PT pore (Haworth and Hunter, 1979; Ichas, F. and Mazat, 1998). The presence of free radicals (*i.e.* oxidative stress) can also cause the PT pore to open (Fiskum, 2001; Brustovetsky et al., 2003).

Factors that cause the PT pore to close or remain closed include acidic conditions (Friberg and Wieloch, 2002), high concentrations of ADP (Brustovetsky et al., 2003; Hunter and Hayworth 1979), high concentrations of ATP (Beutner et al., 1998), and high concentrations of NADH (Hunter and Hayworth, 1979). Divalent cations like $Mg^{2+}$ also inhibit PT because they can compete with $Ca^{2+}$ for the $Ca^{2+}$ binding sites on the matrix side of the PT pore (Hayworth and Hunter, 1979).

Whether the mitochondria resulting from the above PT-inducing stimuli are all equally damaged or whether different stages of damage exist is poorly established. This question is of particular importance with respect to the ongoing discussion about the implication of PT in distinct cell death scenarios (Bernardi et al., 2006; Grimm and Brdiczka, 2007; Kroemer et al., 2007; Green and Kroemer, 2004; Halestrap, 2006; Kinnally and Antonsson, 2007; Tsujimoto and Shimizu, 2007). Substantial mitochondrial damage may cause ATP depletion and necrosis, whereas partial damage or damage in which some mitochondria maintain their membrane potential and produce ATP may drive apoptotic cell death or, if it occurs below a certain threshold level, can even simply provoke the removal of damaged mitochondria by autophagy (Halestrap, 2006; Ott et al., 2007).

In summary, not only the assessment of PT occurrence under *in vivo* conditions but also the extent of mitochondrial damage after PT *in vivo* and *in vitro*, also in the context of various diseases resulting in or from cell death, is still a controversial issue (Bernardi et al., 2006; Bernardi et al., 1999) and so far, no direct method has been established which assesses mitochondrial damage in quantitative terms and which distinguishes between potentially existing mitochondrial subpopulations preferably exhibiting heterogeneous levels of damage (for a summary of applied methods see Galluzzi et al., 2007).

Thus, there is a need for improved methods of detecting mitochondrial damage.

Accordingly, in a first aspect the present invention relates to a method for detecting mitochondrial damage in mammalian tissue or cells comprising: (a) isolating mitochondria from a sample obtained from a mammal and containing tissue or cells containing mitochondria; (b1) separating populations of the isolated mitochondria of step (a) by means of their electrophoretic mobility; and (c1) comparing the populations of the mitochondria obtained in step (b1) with those of a reference sample containing tissue or cells displaying a non-pathological phenotype, wherein a rise in the number of populations, a shift of the population(s) towards the anode or the cathode and/or an increase in the heterogeneity of one or more populations separated in step (b1) as compared to those of the reference sample is indicative of mitochondrial damage; or (b2) analyzing the average surface charge of the isolated mitochondria of step (a); and (c2) comparing the average surface charge determined in step (b2) with that of a reference sample containing cells displaying a non-pathological phenotype, wherein a shift of the average surface charge of the mitochondria analyzed in step (b2) as compared to that of the mitochondria of the reference sample is indicative of mitochondrial damage; and (d) in the case that a shift of the population(s) towards the cathode in step (c1) or a shift of the average surface charge to the positive in step (c2) is detected or, confirming that mitochondrial damage is present.

"Mitochondrial damage" as used in the present invention refers to the mitochondrial outer membrane permeabilization (MOMP). MOMP is either the result of an increase in the permeability of the mitochondrial inner membrane which is called permeability transition (PT). This increase can occur to different extents leading either to reversible mitochondrial damage where only PT is present or to MOMP, the latter comprising different degrees of membrane rupture and mostly leading to cell death. Alternatively, the outer mitochondrial membrane may be targeted and permeabilized directly by a variety of different stimuli (e.g. the protein BAX). The extent of mitochondrial damage is defined by the extent of the mitochondrial inner membrane exposed which may range from minor ruptures or pores (exposure of less than 1 to 10% of the inner membrane) via intermediate rupture (exposure of more than 10%, such as 20, 30 or 40% to up to 50% of the inner membrane) to an almost complete removal of the outer membrane (exposure of more than 50%, such as 60, 70, 80 or 90% to 100% of the inner membrane).

A large number of distinct stimuli can trigger PT (Green and Kroemer, 2004). The increased permeability of solutes towards the matrix space causes an osmotically driven water influx. The concomitant extension of the mitochondrial inner membrane ("swelling") leads to a matrix transition from the "aggregated" to the "orthodox" state and finally results in mitochondrial outer membrane permeabilization (MOMP) (Hunter et al., 1976; Petronilli et al., 1993; Van der Heiden et al., 1997; Brenner and Grimm, 2006). MOMP leads to the release of proteins normally found in the space between the inner and outer mitochondrial membranes (*e.g.* cytochrome c and AIF).

"Isolating mitochondria" as used in connection with the present invention defines the step of obtaining mitochondria to be applied to the separation/analysis step of the method of the present invention from a sample. By isolating the mitochondria, they are separated from the remainder of the cell so that their surface is not covered by cell debris such as fragments of the cell membrane but is exposed and accessible for external influences such as electric fields. The skilled person is aware of methods for isolating mitochondria (for exemplary methods see Pallotti and Lenaz, 2007). An exemplary method is described in Example 1 below.

The term "electrophoretic mobility" determines the extent or velocity of movement of a charged particle in a given electric field. It depends on several physical parameters, in particular on the surface charge, but also on the size of the particles. The driving force F causing the movement of the particles is the force acting on a particle having a charge q within an electrical field with a given field force E

$$F = q \cdot E$$

The force acting to the contrary results from the viscosity $\eta$ and the particle size ($6 \cdot \pi \cdot \gamma$, as idealized for spherical particles) and can be calculated according to Stokes law:

$$F = 6 \cdot \pi \cdot r \cdot \eta \cdot \nu$$

The above equations result in the theoretical electrophoretic mobility $\left(\mu_{e,p}^{0}\right)$ for an idealized state without a carrier and with practically salt-free electrolytes.

$$\mu_{e,p}^{0} = \frac{\nu}{E} = \frac{q}{6 \cdot \pi \cdot r \cdot \eta}$$

In real systems, factors such as the hydrate shell, the ion atmosphere, the grade of dissociation of the electrolyte and effects caused by the carrier (molecular sieve, electro-osmosis and adsorption effects) also influence the electrophoretic mobility.

Throughout the present invention the term "sample" denotes a sample from a human, a rodent, a ruminant or any mammal commonly used in laboratory experiments. A sample can be liquid or solid and contains cells from a tissue or organ.

In connection with the first aspect of the present invention, a sample to be analyzed has undergone or is at least suspected of having undergone mitochondrial damage and has a pathological phenotype. In contrast to a non-pathological phenotype described further below, a pathological phenotype is **characterized in that** it exhibits symptoms of a pathological condition caused e.g. by unusual stress such as oxidative stress, inflammation or infection.

In the context of the present invention, the term "populations of mitochondria", interchangeably used with "subpopulations of mitochondria" denotes groups of mitochondria which differ by their electrophoretic mobility or surface charge or both. Depending on the extent of permeability transition or MOMP which the mitochondria undergo, these properties change resulting in populations or subpopulations of mitochondria to which a certain degree of damage can be assigned.

The term "reference sample" as used in connection with this aspect of the present invention denotes a sample from the same or a different individual as the one from which the sample to be analyzed was taken. If a sample from the same individual is used as a reference sample, it can be taken from an undamaged part of the same tissue or organ as the sample to be measured or from a different tissue or organ with a reaction in response to stimuli which is comparable to that of the tissues or organs the sample was taken from and/or with comparable properties as regards susceptibility to substances causing mitochondrial damage. Alternatively, the reference sample can be taken from the same individual from the same tissue or organ before the damage happens, resulting in a direct comparison of the distribution of mitochondrial subpopulations before and after the damage. For example, in the case of an individual in danger of undergoing mitochondrial damage in one or more tissues or organs, a sample of the organ(s) or tissue(s) to be treated or otherwise in danger of being harmed can be taken prior to the operation and another one after the operation. A particular example describes an operation wherein a heart-lung-machine (HLM) is used. Here, the method of the present invention can be used to determine mitochondrial damage caused following ischemia/reperfusion (ischemia/reperfusion disease).

Alternatively, the reference sample can be taken from a different individual. In this regard, the reference sample is preferably taken from the same tissue or organ as the sample to be analyzed. The individual is preferably from the same species as that from which the sample to be measured was obtained.

The reference sample does not necessarily have to be prepared simultaneously to the sample. It may well be stored under preserving conditions, e.g. frozen, prior to the sample analysis. In the case of analyzing the average surface charge, the reference sample may well be separated and/or analyzed prior to the sample analysis and the results of the analysis may be stored electronically or otherwise and be retrievable upon request.

A "non-pathological phenotype" in the context of the present invention relates to the apparent state of the cells of the reference sample as normal, i.e. they display a phenotype which is to be expected from a sample which is not affected by a pathological condition, in particular has not undergone any unusual stress such as oxidative stress, inflammation, infection or is concerned with other diseases resulting in an altered metabolism or an altered state of the mitochondria, in particular an altered mitochondrial metabolism or mitochondrial damage.

In accordance with the present invention, a new approach to assess mitochondrial damage, in particular PT-induced MOMP or MOMP induced directly by different stimuli (as described above), was found which is based on the different surface charge of the inner and outer mitochondrial membrane of isolated mitochondria from higher eukaryotes. In 1970, Heidrich et al. reported that inner membrane-matrix vesicles (IMV) generated from intact rat liver mitochondria display a stronger deflection in an electrical field towards the anode when compared to whole mitochondria. The experiments carried out were not remotely related to PT or PT-induced MOMP or a disease causing any of the two and were thus not related to the present invention. The inventors recently reported that zone electrophoresis in a free flow device could be useful for the preparative analysis of yeast mitochondria (Zischka et al., 2006) which differ significantly from mammalian mitochondria in terms of (protein-) composition, biological functions and organization. Furthermore, important differences were found between the electrophoretic deflection behaviour of yeast mitochondria and mitochondria isolated from mammalian systems. Based on the current knowledge, electrophoretic deflection of mammalian mitochondria is primarily dependent on the surface charge whereas the electrophoretic deflection of yeast mitochondria is dependent on several other aspects like hydrodynamic properties. Importantly, the study of the electrophoretic deflection of yeast mitochondria was not remotely related to PT or PT-induced MOMP or a disease causing any of the two and is thus not related to the present invention. Especially an assessment of mitochondrial damage in the form of MOMP, which is the focus of the present invention, was not investigated in the reported study. Thus the skilled person would and could not have concluded from the reported results of yeast mitochondria on the electrophoretic deflection behaviour of mammalian mitochondria nor on the electrophoretic deflection of mammalian mitochondria heterogeneously damaged by MOMP.

The inner surface of rat liver mitochondria is more negatively charged than the surface of their enveloping outer membranes. Upon MOMP, the total surface charge of mitochondria is altered due to the simultaneous exposure of the differently charged outer and inner mitochondrial membranes. Accordingly, by detecting differences in the total charges of mitochondria due to the additional exposure of inner charges, the method of the present invention can detect not only whether mitochondria have undergone (PT- or directly induced) MOMP but also to which extent mitochondrial damage has occurred or whether this process is still reversible. As shown in the examples, up to four distinct mitochondrial populations (M0 to M3) emerge upon $Ca^{2+}$-induced PT which can be detected by analyzing their electrophoretic mobility and/or surface charge, e.g. by zone electrophoresis in a free flow device and other suitable means to separate mitochondria by charge differences. The relative amount and appearance of each of these populations is dependent either on the dose of the inducer of PT or the duration of induction. In this regard, a (quantitative) transition towards severely damaged organelles at higher doses or prolonged induction occurs in the form of a rise in the number of populations, a shift/transition of the populations towards the anode and/or an increase in the heterogeneity of one or more populations as compared to a reference sample. An increased heterogeneity of one or more populations means that the variance within one population increases resulting, inter alia, in bigger differences in the surface charge or the electrophoretic mobility. The profile obtained by analyzing the electrophoretic mobility of mitochondria by electrophoretic means and the breadth of the peaks observed therein displaying different degrees of heterogeneity directly indicates whether an increase in the heterogeneity has taken place. The examples disclosed in the present invention do not only show that *in vitro* induced PT/MOMP can be detected but also that MOMP directly arises upon ischemia/reperfusion. These results show that the findings in context of the present invention can be applied for research as well as for diagnostic purposes.

PT or PT-induced MOMP may also result in a more positive net surface charge as compared to unaffected mitochondria in a reference sample which is manifested in a transition or shift of the subpopulations towards the cathode. Inter alia, upon exposure to heavy metal ions, these ions can either oxidize the negative charges on the exposed surface of the mitochondria or the ions themselves attached to the surface can cause the shift to a more positive net charge. This effect has to be distinguished from that expected upon a regeneration of the mitochondria. Accordingly, if a shift or transition of the mitochondrial subpopulations towards the cathode is observed, a further method step has to be applied that serves to confirm that mitochondrial damage rather than an amelioration of the mitochondrial condition is present. Suitable means to confirm the status of the mitochondria or mitochondrial subpopulation(s), respectively, are described further below. As an exemplary means, the detection of proteins usually not exposed on the outer surface of mitochondria by e.g. immunological means can be applied.

In summary, the present method provides means for the direct and quantitative analysis of the status of mitochondria. The extent of mitochondrial damage detected is directly related to the number, the position and the heterogeneity of the subpopulations detected and can give rise to the status of the individual the sample was taken from or to a prognosis of this state. The findings of the present invention and the method resulting herefrom can be applied in the analysis of disease states or conditions as will be described further below.

In a second embodiment, the present invention relates to a method of identifying an agent causing mitochondrial damage in mammalian cells comprising: (a) contacting with a test agent (i) a non-human animal and obtaining a sample containing tissue or cells to be analysed; or (ii) a sample obtained from a mammal and containing cells with mitochondria; or (iii) cultured cells containing mitochondria; or (iv) isolated mitochondria; and isolating the mitochondria from the sample of (i), (ii) or the cells of (iii); (b1) separating populations of the isolated mitochondria of step (a) by means of their electrophoretic mobility; and (c1) comparing the populations of the mitochondria obtained by analysing the electrophoretic mobility in (b1) with those of an untreated sample, wherein a shift of the population(s) towards the anode or the cathode, a rise in the number of mitochondrial populations and/or an increase in the heterogeneity of one or more populations separated in step (b1) as compared to the untreated sample is indicative of mitochondrial damage caused by the agent; or (b2) analyzing the average surface charge of the isolated mitochondria of step (a); and (c2) comparing the average surface charge determined in step (b2) with that of an untreated sample, wherein a shift of the average surface charge of the mitochondria analyzed in step (b2) as compared to that of the untreated sample is indicative of mitochondrial damage caused by the agent; and (d) in the case that a shift of the population(s) towards the cathode or a shift of the average surface charge to the positive is detected, confirming that mitochondrial damage caused by the agent is present

An "untreated sample" as used in connection with the present invention can be a sample from the same source, i.e. from the same or a different tissue or organ of the same individual which has been obtained prior to the contacting step with the agent to be tested. In case of a different tissue or organ, it exerts a reaction in response to stimuli which is comparable to that of the tissues or organs the sample was taken from and/or have comparable properties as regards susceptibility to substances causing mitochondrial damage. If no such untreated sample is available, a suitable sample can also be obtained from a different individual, preferably of the same species, which was kept under the same conditions.

An "agent causing mitochondrial damage" can directly or indirectly exert this effect. Certain agents act directly on the mitochondria and cause PT or MOMP (e.g. viral proteins, reactive oxygen species (ROS), actractyloside, acetaminophen, ethanol, heavy metals; for a detailed list see Table 5S and 6S in Green and Kroemer, 2004), whereas others interfere with signal transduction pathways influencing mitochondria. Examples of such signal transduction pathways are apoptotic pathways or those resulting in an excess $Ca^{2+}$ concentration in the cell. Accordingly, in this aspect of the present invention, only agents directly damaging mitochondria can be detected using isolated mitochondria, whereas indirectly acting agents can be identified if samples containing cells or tissue are exposed to these agents in addition.

"Contacting a non-human animal" can be effected in various different ways. Nonlimiting examples are feeding, injection, application onto the skin or other parts of the body.

This second aspect of the present invention basically relies on the same finding and principles as described above for the method of detecting mitochondrial damage with a different purpose, *i.e.* the identification of agents causing mitochondrial damage.

In a third embodiment, the present invention relates to a method of identifying an agent capable of regenerating damaged mammalian mitochondria comprising: (a) contacting with a test agent (i) a non-human animal containing cells with damaged mitochondria and obtaining a sample containing tissue or cells to be analyzed; or (ii) a sample obtained from a mammal and containing cells with damaged mitochondria; or (iii) cultured cells containing damaged mitochondria; or (iv) isolated damaged mitochondria; and isolating the mitochondria from the sample of (i), (ii) or the cells of (iii); (b1) separating populations of the isolated mitochondria of step (a) by means of their electrophoretic mobility; and (c1) comparing the populations of the mitochondria obtained in (b1) by analysing the electrophoretic mobility with those of a reference sample not contacted with the test agent and containing damaged mitochondria, wherein a shift of the mitochondrial populations towards the cathode, a change in the distribution of the mitochondrial populations and/or a decrease in the heterogeneity of one or more populations separated in step (b1) as compared to the reference sample is indicative of a regeneration of the mitochondria caused by the agent; or (b2) analyzing the average surface charge of the isolated mitochondria of step (a); and (c2) comparing the average surface charge determined in step (b2) with that of a reference sample not contacted with the test agent and containing damaged mitochondria, wherein a shift of the average surface charge of the mitochondria analyzed in step (b2) as compared to that of the reference sample is indicative of a regeneration of the mitochondria caused by the agent; and (d) in the case that a shift of the population (s) towards the anode or a shift of the average surface charge to the negative is detected, confirming that the mitochondria have regenerated.

Capable of regenerating damaged mammalian mitochondria denotes the ability of an agent to reverse damage or ameliorate the state of mitochondria regarding their functional and structural intactness.

A reference sample as used in connection with this aspect of the present invention refers to a sample from the same ("the same source") or a different individual as the one contacted with the test agent. In this aspect of the invention, the reference sample contains damaged mitochondria and has not been contacted with the agent to be tested. If necessary, the state of the mitochondria of the reference sample can be determined by comparison with those in another reference sample displaying a non-pathological phenotype. The damage can, inter alia, be caused by ageing or stress, e.g. oxidative stress or intoxication with mitochondria damaging agents.

If a sample from the same individual is used as a reference sample, it can be taken from the same tissue or organ as the sample to be measured or from a different tissue or organ with comparable properties as regards susceptibility to substances having regenerative capabilities.

Alternatively, the reference sample can be taken from a different individual. In this regard, the reference sample is preferably taken from the same tissue or organ as the sample to be analyzed. The individual is preferably from the same species as that from which the sample to be measured was obtained.

This aspect of the present invention relies on the assumption that certain agents can have a regenerative effect on damaged mitochondria. Accordingly, by detecting differences in the total charges of mitochondria due to the additional exposure of inner charges, the method of the present invention can detect whether test agent have a regenerative effect on mitochondria. The relative amount and appearance of each of the mitochondrial populations described in the context of the other aspects of the present invention is assumed to be dependent either on the dose of the potentially regenerative agent or the duration of exposure of the mitochondria or the sample analyzed to the agent. In this regard, a (quantitative) transition towards regenerated organelles at higher doses or prolonged exposure is assumed to occur in the form of a shift/transition of the populations towards the cathode, a change in the distribution of the mitochondrial populations and/or a decrease in the heterogeneity of one or more populations as compared to a reference sample. A decreased heterogeneity of one or more populations means that the variance within one population decreases resulting, inter alia, in smaller differences in the surface charge or the electrophoretic mobility. The profile obtained by analyzing the electrophoretic mobility of mitochondria by electrophoretic means and the breadth of the peaks observed therein directly indicates whether such a decrease in the heterogeneity has taken place.

A regeneration of mitochondria may also result in a more negative net surface charge as compared to unaffected mitochondria in a reference sample which is manifested in a transition or shift of the subpopulations towards the anode. Inter alia, if the mitochondria had been exposed to heavy metal ions followed by the exposure to an agent with regenerative capabilities, the more positive net charge caused by the heavy metal ions is counteracted through regeneration of the mitochondria. Without wishing to be bound to any theory, the Applicant assumes that mechanisms exist causing the removal or masking of these positively charged ions. Accordingly, if a shift or transition of the mitochondrial subpopulations towards the anode is observed, a further method step has to be applied that serves to confirm that regeneration of mitochondria rather than mitochondrial damage is present. Suitable means to confirm the status of the mitochondria or mitochondrial subpopulation(s), respectively, are described further below. As an exemplary means, the functional state of mitochondria may be assessed *e.g.* by respiratory measurements.

Unless stated otherwise, preferred embodiments relate to all of the above methods disclosed in the present invention.

In a preferred embodiment, the methods of the present invention further comprise separating the mitochondria according to their electrophoretic mobility directly prior to the analysis of the average surface charge. Accordingly, the resulting populations are individually subjected to the analysis of the average surface charge. In this embodiment of the present invention, the reference sample or untreated sample has to be treated accordingly, i.e. the mitochondria have to be separated according to their electrophoretic mobility prior to the analysis of the average surface charge in order to provide suitable means for comparison.

This additional method step will result in a clearer difference between the results obtained for the sample and those for the reference sample or the untreated sample since the average surface charge of isolated and thus homogenous subpopulations is analysed.

In another preferred embodiment, the separation according to the electrophoretic mobility is effected by free-flow electrophoresis.

Free flow electrophoresis is a process in which a sample stream is introduced into a liquid buffer flow within a separation vessel. A fixed or varying electric field is maintained across the separation vessel perpendicular to the buffer flow. Species of virtually identical biomolecules or bioparticles with different surface charges thus react or move differently within electric fields. Passing a selected sample of biomolecules or bioparticles through an electric field in an appropriate buffer or carrier results in the molecules migrating to separate positions or zones in the electrical field representative of their charge. Thus, visually and chemically similar biomolecules or bioparticles can be separated into subspecies according to their electrophoretic mobility. Individual components in the sample stream are separated from each other on the basis of their mobility in the imposed electric field and are collected at the exit of the separation vessel in one or several collection vessels (Hannig and Heidrich, 1990; Krivankova and Bocek, 1998).

Over the time, different varieties of FFE have been developed.

The continuous zone electrophoresis (ZE-) FFE separation technique is based on the difference between the electrophoretic mobility of the particles to be separated. ZE-FFE enables for the isolation of analytes and particles on the basis of differing size and/or shape and/or net surface charge. The ZE-FFE method is especially suitable for separating sensitive bioparticles and complexes when specific demands have to be met by the separation medium during separation. This is particularly the case when the biological function and integrity of the particles and/or the original state of the particles prior to removal as a sample have to be maintained following separation. The special requirements in these cases are a very restricted pH range for the separation medium, a good separation medium buffer capacity, physiological compatibility of the buffer substances used and a minimum content of various essential cations and anions *etc.*

Other commonly utilized FFE varieties are continuous isoelectric focusing (CHIEF) FFE separation and continuous isotachophoresis (CITP) FFE separation.

Various combinations of the above separation techniques (e.g. CHIEF and ZE FFE or CHIEF and CITP) are possible. In this way, it is *e.g.* possible to use different separation media at the same time in the separation chamber of the FFE apparatus, employing different separation parameters simultaneously.

For immuno-FFE, specific antibodies are coupled to defined surface epitopes of cells or organelles, *e.g.* mitochondria, to be separated. The altered net charge of the particle to be analyzed created thereby effects an altered electrophoretic mobility according to the presence of the surface epitopes on the particles (see *e.g.* Hansen and Hannig (1982)).

In another preferred embodiment, the surface charge is analyzed by determining the zeta potential.

The zeta potential is the electric potential in the interfacial double layer at the slipping plane of a mobile particle in a suspension. The electric potential describes the ability of an electric field caused by a charge to transfer power to other charges.

The interfacial double layer is a structure that appears on the surface of an object when it is placed into a liquid. This object may be a solid particle, gas bubble or liquid droplet. The interfacial double layer is usually important when the size of the object is very small, on the scale of micrometers or even nanometers.

The structure of the interfacial double layer consists of two parallel layers of ions. One layer which is either positive or negative coincides with the surface of the object. The other layer is located in the fluid. This second layer electrically screens the first one. It is diffuse because it forms under the influence of electric attraction and thermal motion of free ions in fluids. Double layers exist in practically all heterogeneous fluid based systems, such as blood, protein solutions or suspensions containing mitochondria. One class of phenomena arising from the existence of the interfacial double layer is electrokinetic phenomena.

In electrokinetic phenomena, the influence of an external force on the diffuse layer generates tangential motion of a fluid with respect to an adjacent charged surface. This force may be electric, a pressure gradient, a concentration gradient or gravity. In addition, the moving phase may be either a continuous fluid or a dispersed phase.

Various combinations of the driving force and moving phase determine various electrokinetic effects. The family of electrokinetic phenomena includes: electrophoresis (the motion of particles under the influence of an electric field); electro-osmosis (the motion of liquid in a porous body under the influence of an electric field); diffusiphoresis (the motion of particles under the influence of the chemical potential gradient); capillary osmosis (the motion of liquid in a porous body under the influence of the chemical potential gradient) and sedimentation potential (the electric field generated by sedimenting colloid particles).

Due to the above described relationship between the surface charge and the electrophoretic mobility, with the determination of any of the two parameters the other can be calculated.

In a preferred embodiment of the present invention relating to the method of identifying an agent causing mitochondrial damage in mammalian cells, the non-human animal, the sample, the cells or the mitochondria, prior to contacting with the test agent, and the untreated sample of step (c1) or (c2), each display a non-pathological phenotype.

In another preferred embodiment of the present invention relating to the method of identifying an agent causing mitochondrial damage in mammalian cells, the untreated or reference sample of step (c1) or (c2) and the sample, the cells or the mitochondria of step (a) are from the same source. The term "same source" denotes that the sample and the untreated or reference sample are from the same individual. The untreated or reference sample can either be taken from the same tissue or organ of the individual as the sample to be measured or from a different tissue or organ with comparable properties as regards susceptibility to substances having regenerative capabilities.

In a preferred embodiment of the method for detecting mitochondrial damage in mammalian tissue or cells or the method of identifying an agent causing mitochondrial damage in mammalian cells, in the case that a shift of the population(s) towards the anode in step (c1) or a shift of the average surface charge to the negative in step (c2) is detected, the method further comprises confirming that mitochondrial damage is present. Whereas a confirmation step is necessary in the case that a shift of the population(s) to the cathode or a shift of the average surface charge to the positive is detected, such a step is not necessary if a shift of the population(s) to the anode or a shift of the average surface charge to the negative is detected since the latter result is expected in the majority of the examined cases. On the contrary, a shift of the population(s) to the cathode or a shift of the average surface charge to the positive as observed e.g. upon exposure to heavy metal ions, only constitutes a minority of cases.

In another preferred embodiment of the method for identifying an agent capable of regenerating damaged mitochondria, in the case that a shift of the population(s) towards the cathode in step (c1) or a shift of the average surface charge to the negative in step (c2) is detected, the method further comprises confirming that the mitochondria have regenerated. The reasons given in the preceding paragraph for a confirmation step in case the results correspond to the majority of expected cases also apply to this embodiment.

In a further preferred embodiment of the method relating to the identification of an agent capable of regenerating damaged mammalian mitochondria said method further comprises: (e1) comparing the electrophoretic mobility of the populations of the mitochondria obtained in (b1) with that of populations of a reference sample displaying a non-pathological phenotype, wherein a position, distribution and/or a heterogeneity of the mitochondrial populations similar to one or more of those of the reference sample is indicative of a regeneration of the mitochondria caused by the agent; or (e2) comparing the average surface charge determined in step (b2) with that of a reference sample displaying a non-pathological phenotype, wherein an average surface charge similar to that of the reference sample is indicative of a regeneration of the mitochondria caused by the agent. Steps (e1) and (e2) can be carried out at any time after steps (b1) or (b2).

Step (e1) or (e2) serves to confirm the results obtained in steps (c1), (c2) or (d) by applying the approach that the electrochemical profile or surface charge of mitochondria which have regenerated should resemble those of a reference sample displaying a non-pathological phenotype. This step is, however, not applicable if only a partial regeneration has taken place in the sample since in this case the profiles or surface charges of the sample and the reference sample displaying a non-pathological phenotype are not necessarily similar.

In a fourth aspect, the present invention relates to a method of identifying an agent capable of regenerating damaged mammalian mitochondria comprising: (a) contacting with a test agent (i) a non-human animal containing cells with damaged mitochondria and obtaining a sample containing tissue or cells to be analyzed; or (ii) a sample obtained from a mammal and containing cells with damaged mitochondria; or (iii) cultured cells containing damaged mitochondria; or (iv) isolated damaged mitochondria; and isolating the mitochondria from the sample of (i), (ii) or the cells of (iii); (b1) separating populations of the isolated mitochondria of step (a) by means of their electrophoretic mobility; and (c1) (corresponding to step (e1) above) comparing the electrophoretic mobility of the populations of the mitochondria obtained in (b1) with that of populations of a reference sample displaying a non-pathological phenotype, wherein a position, distribution and/or a heterogeneity of the mitochondrial populations similar to one or more of those of the reference sample is indicative of a regeneration of the mitochondria caused by the agent; or (b2) analyzing the average surface charge of the isolated mitochondria of step (a); and (c2) (corresponding to step (e2) above) comparing the average surface charge determined in step (b2) with that of a reference sample displaying a non-pathological phenotype, wherein an average surface charge similar to that of the reference sample is indicative of a regeneration of the mitochondria caused by the agent; and (d) in the case that a shift of the population(s) towards the anode or a shift of the average surface charge to the negative is detected, confirming that the mitochondria have regenerated.

The term "reference sample displaying a non-pathological phenotype" as used in connection with this aspect of the present invention and the preceding preferred embodiment denotes a sample from the same or a different individual as the one from which the sample to be analyzed was taken. If a sample from the same individual is used as a reference sample, it can be taken from an undamaged part of the same tissue or organ as the sample to be measured or from a different tissue or organ with comparable properties as regards susceptibility to test agents. Alternatively, the reference sample can be taken from a different individual. In this regard, the reference sample is preferably taken from the same tissue or organ as the sample to which the test agent was applied. The individual is preferably from the same species as that from which the sample to be measured was obtained.

In a further preferred embodiment of the present invention, the mitochondrial damage is mitochondrial outer membrane permeabilization (MOMP).

As described above, beside a direct damaging attack on the outer mitochondrial membrane, MOMP is the culmination of a sequence of events starting with an increase in the permeability of the inner mitochondrial membrane. Under normal conditions, the inner mitochondrial membrane is quasi-impermeable for small molecules, allowing the generation of the electrochemical gradient which is indispensable for mitochondrial function. In the course of MOMP, PT (permeability transition) pores form. The exact molecular composition of PT pores is not known: it is suggested in the art that at the inner mitochondrial membrane the adenine nucleotide translocator (ANT) is involved in PT pore formation, in association with several molecules of the outer mitochondrial membrane such as the peripheral benzodiazepine receptor and the voltage-dependent anion channel. ANT ligands such as atractyloside (Atr) and bongkrekic acid (BA) enhance or reduce the probability of PT pore opening, respectively. The PT pore functions as a voltage sensor, as thiol sensor and as a sensor of Calcium-Ions: Matrix $Ca^{2+}$ ions increase, whereas external $Ca^{2+}$ ions decrease the probability of pore opening. It is suggested in the art that periodic reversible PT pore opening allows for the release of calcium from the mitochondrial matrix, thus allowing the maintenance of calcium homeostasis. As described more generally above, the opening of PT pores results in a sudden permeability increase of the inner mitochondrial membrane to solutes with a molecular weight of 1500 Da and some proteins, thereby disrupting the deltaPSI (the mitochondrial transmembrane potential) and associated mitochondrial functions. In isolated mitochondria, PT is accompanied by colloidosmotic swelling and uncoupling of oxidative phosphorylation, as well as by the loss of low molecular weight matrix molecules such as calcium and glutathione. The concomitant extension of the mitochondrial inner membrane ("swelling") leads to a matrix transition from the "aggregated" to the "orthodox" state and finally results in MOMP.

In another preferred embodiment of the present invention, the confirmation that mitochondrial damage caused by the agent or a regeneration of the mitochondria caused by the agent is present comprises determining the presence and optionally the quantity of proteins specific for the outer and/or inner mitochondrial membrane and/or for the inter membrane space in one or more of the populations.

The determination of proteins in this step of the method of the present invention describes a qualitative as well as a quantitative characterization of the proteins present on the outer and/or inner mitochondrial membrane and/or for the inter membrane space.

Examples for well-known assays based on qualitative and/or quantitative protein detection include without limitation method steps such as ion exchange chromatography, gel filtration chromatography, affinity chromatography, high pressure liquid chromatography (HPLC), reversed phase HPLC, disc gel electrophoresis, two-dimensional gel electrophoresis, optionally in combination with mass spectrometric methods for protein identification, liquid chromatographic methods, optionally in combination with mass spectrometry, immunoblotting, immunoprecipitation, immune electrophoresis and enzymatic activity assays. Typical references for these methods appear in specialized textbooks like the "Methods in Molecular Biology" or "Methods in Enzymology" series apart from single or review articles (*e.g.* Rabilloud (2002)).

In another preferred embodiment of the present invention relating to a method for detecting mitochondrial damage in mammalian tissue or cells or a method of identifying an agent causing mitochondrial damage in mammalian cells, the mitochondrial damage detected results in cell death or regeneration/survival of the cells.

Cell death can arise via apoptosis or necrosis, which are usually considered separated processes. Recently, the view has emerged that both are frequently the consequence of the same initiating factors and signalling pathways (for a review of the role of mitochondrial permeability transition in cell death see Tsujimoto and Shimizu, 2006).

Necrosis or oncotic necrosis is typically the consequence of acute metabolic perturbation as occurs *e.g.* in ischemia/reperfusion or acute drug-induced toxicity (*e.g.* acetaminophen toxicity), frequently leading to ATP depletion. The major role of mitochondria in most cells is the provision of ATP by oxidative phosphorylation to drive energy-dependent processes. To fulfil this function, mitochondria must maintain a membrane potential. This demands that their inner membrane remains impermeable to most kinds of ions and metabolites for which specific transport mechanisms are present. One ion for which specific transport mechanisms exist is the $Ca^{2+}$ ion, which enters via an electrogenic uniporter, now known to be a channel, and is pumped out again by an $Na^+/Ca^{2+}$ antiporter. Whereas the activity of the $Na^+/Ca^{2+}$ antiporter saturates as mitochondrial matrix $Ca^{2+}$ increases, the uniporter acts as a channel and is thus not saturated with increasing extramitochondrial $Ca^{2+}$ concentration. Consequently, as the extramitochondrial $Ca^{2+}$ concentration increases beyond a certain value, the mitochondria can no longer regulate their matrix $Ca^{2+}$ concentration, and mitochondrial overload ensues. In some mitochondria under normal conditions, this overload can occur without damage to the mitochondria. However, when the overload is also accompanied by a combination of other factors, most notably oxidative stress, high phosphate concentrations and low adenine nucleotide concentrations, the mitochondria undergo PT leading to PT pore opening. The resulting permeability includes protons, and thus the mitochondria become uncoupled. Since a pH gradient or membrane potential can no longer be maintained, the mitochondria not only become incapable of ATP synthesis, but also now actively degrade ATP. Without further action, this will inevitably lead to a loss of metabolic and ionic integrity of the cells, and ultimately to cell death by necrosis as damage by degradative enzymes goes unrepaired (reviewed in Halestrap (2006); for a further review see Malhi et al. (2006)).

In contrast to necrosis, the process of apoptosis is controlled by a diverse range of cell signals which may originate either extracellularly (*extrinsic inducers*) or intracellularly (*intrinsic inducers*). Extracellular signals may include hormones, growth factors, nitric oxide or cytokines, and therefore must either cross the plasma membrane or transduce to effect a response. These signals may positively or negatively induce apoptosis; in this context the binding and subsequent initiation of apoptosis by a molecule is termed positive, whereas the active repression of apoptosis by a molecule is termed negative.

Intracellular apoptotic signalling is a response initiated by a cell in response to stress, and may ultimately result in cell suicide. The binding of nuclear receptors by glucocorticoids, heat, radiation, nutrient deprivation, viral infection and hypoxia are, inter alia, factors which can lead to the release of intracellular apoptotic signals by a damaged cell. Before the actual process of cell death is carried out by enzymes, apoptotic signals must be connected to the actual death pathway by way of regulatory proteins. This step allows apoptotic signals to either culminate in cell death, or be aborted should the cell no longer need to die resulting in survival or regeneration of the cell. Several proteins are involved, however two main methods of achieving regulation have been identified: targeting mitochondria functionality, or directly transducing the signal via *adapter proteins* to the apoptotic mechanisms. The whole preparation process requires energy and functioning cell machinery.

Apoptotic proteins which target mitochondria affect them in different ways; they may cause mitochondrial swelling through the formation of membrane pores, or they may increase the permeability of the mitochondrial membrane and cause apoptotic effectors to leak out. There is also a growing body of evidence which indicates that nitric oxide (NO) is able to induce apoptosis by helping to dissipate the membrane potential of mitochondria and therefore make it more permeable. Mitochondrial proteins known as SMACs (second mitochondria-derived activator of caspases) are released into the cytosol following an increase in permeability. SMAC binds to *inhibitor of apoptosis proteins* (IAPs) and deactivates them, preventing the IAPs from arresting the apoptotic process and therefore allowing apoptosis to proceed. IAP also normally suppresses the activity of caspases, which carry out the degradation of the cell, therefore the actual degradation enzymes can be seen to be indirectly regulated by mitochondrial permeability.

Cytochrome c is also released from mitochondria due to increased permeability of the outer mitochondrial membrane, and serves as regulatory factor as it precedes morphological change associated with apoptosis. Once cytochrome c is released it binds to *Apaf-1* and ATP, which then bind to *pro-caspase-9* to create a protein complex known as an apoptosome. The apoptosome cleaves the pro-caspase to its active form of caspase-9, which in turn activates the effector caspase-3.

The mitochondrial permeability is itself subject to regulation by various proteins, such as those encoded by the mammalian *Bcl-2* gene family of pro- and anti-apoptopic genes, the homologs of the *ced-9* gene found in *C. elegans. Bcl-2* proteins are able to promote (e.g. BAX, BID, BAK or BAD) or inhibit (e.g. Bcl-XI or Bcl-2) apoptosis by either direct action on mitochondrial permeability, or indirectly through other proteins. Importantly, the actions of some *Bcl-2* proteins are able to halt apoptosis even if cytochrome c has been released by the mitochondria. Thus, e.g. test agents capable of regenerating damaged mitochondria may act by increasing the expression and/or activity of these proteins.

The many different types of apoptotic pathways utilize a multitude of different biochemical components, many of them not yet understood. As a pathway is more or less sequential in nature; removing or modifying one component leads to an effect in another. In a living organism the effects arising are often expressed in the form of a disease or disorder. The concept overlying each disease or disorder is the same: the normal functioning of the pathway has been disrupted in such a way as to impair the ability of the cell to undergo normal apoptosis. This results in a cell which lives past its "use-by-date" and is able to replicate and pass on any faulty machinery to its progeny, increasing the likelihood of the cell becoming cancerous or diseased. Furthermore, enhanced apoptosis can be the result of various non-cancerous diseases.

The events leading to cytochrome *c* release typically cause global mitochondrial dysfunction with mitochondrial membrane depolarization and uncoupling of oxidative phosphorylation. For this reason, the same mechanisms that promote cytochrome *c* release and apoptosis can cause ATP depletion-dependent necrotic cell killing. Although apoptosis and oncotic necrosis have been considered distinct and independent phenomena, the two modes of cell death frequently coexist e.g. in liver pathology, which has led to spirited debates as to what mode of cell death is actually occurring in a given circumstance. An alternative view is that necrosis and apoptosis are interdependent phenomena resulting from activation of shared pathways and signals. Thus, an admixture of necrosis and apoptosis may be expected to occur in many pathophysiological settings.

In an additional preferred embodiment of the present invention, the mitochondrial damage or condition of the mitochondria is indicative of a disease state or condition.

In a more preferred embodiment, the disease state or condition is ischemia/reperfusion damage or injury, a neurodegenerative disease, a liver disease, cancer, a haematological disease or a viral infection.

"Ischemia/reperfusion damage or injury" refers to damage to tissue caused when blood supply returns to the tissue after a period of ischemia. The absence of oxygen and nutrients from blood creates a condition in which the restoration of circulation results in inflammation and oxidative damage through the induction of oxidative stress rather than restoration of normal function. The damage of reperfusion injury is due in part to the inflammatory response of damaged tissues. White blood cells carried to the area by the newly returning blood release a host of inflammatory factors such as interleukins as well as free radicals in response to tissue damage. The restored blood flow reintroduces oxygen within cells that damages cellular proteins, DNA, and the plasma membrane. Damage to the cell's membrane may in turn cause the release of more free radicals. Such reactive species may also act indirectly in redox signaling to turn on apoptosis. Leukocytes may also build up in small capillaries, obstructing them and leading to more ischemia. Reperfusion injury plays a part in the brain's ischemic cascade, which is involved in stroke and brain trauma. Repeated bouts of ischemia and reperfusion injury also are thought to be a factor leading to the formation and failure to heal of chronic wounds such as pressure sores and diabetic foot ulcers. Continuous pressure limits the blood supply and causes ischemia, and the inflammation occurs during reperfusion. As this process is repeated, it eventually damages tissue enough to cause a wound.

Neurodegenerative disorders wherein apoptosis plays a significant role are e.g. Alzheimer's (AD), Parkinson's (PD), Huntington's (HD) diseases, amyotrophic lateral sclerosis (ALS), spinal muscular atrophy (SMA), and diabetic encephalopathy.

The traditional formulation of the amyloid hypothesis as a cause for AD points to the cytotoxicity of mature aggregated amyloid fibrils, which are believed to be the toxic form of the protein responsible for disrupting the cell's calcium ion homeostasis and thus inducing apoptosis.

One example of a liver disease is hepatic dysfunction which is a common clinical complication in malaria, although its pathogenesis remains largely unknown. Malarial infection induces hepatic apoptosis through augmentation of oxidative stress. Apoptosis furthermore plays a significant role in the course of hepatocyte death in acute and chronic hepatitis.

A further, clinically relevant liver disease is acetaminophen hepatotoxicity (Masobuchi et al, 2005; reviewed in Malhi et al., 2006). The hepatotoxicity of acetaminophen (also known as paracetamol) serves as an example of the interrelationship of necrotic and apoptotic cell killing and their common origin in mitochondrial dysfunction (see above). Although safe at therapeutic levels, acetaminophen overdose is the most frequent cause of acute drug-induced liver failure in the United States. Cytochromes P450s metabolize acetaminophen to the reactive metabolite, N-acetyl-p-benzoquinone imine (NAPQI), which reacts with and depletes glutathione, forms covalent adducts and initiates mitochondrial oxidative stress. These events lead to onset of the MPT, and cyclosporin A protects against acetaminophen toxicity both *in vitro* and *in vivo*. As in other forms of liver injury, the roles played by oncotic necrosis and apoptosis in acetaminophen-induced liver damage have been controversial. Studies in mouse hepatocytes show that both modes of cell killing can predominate after acetaminophen exposure. When acetaminophen causes profound ATP depletion, ATP depletion-dependent necrotic cell killing ensues. However, when fructose and glycine are used to prevent ATP depletion, necrosis is blocked, whereas caspase-dependent apoptosis increases. Both with and without fructose plus glycine, mitochondrial inner membrane permeabilization (MPT) occurs, and cyclosporin A decreases both the necrotic and the apoptotic modes of cell killing. Thus, acetaminophen toxicity is one more example of necrapoptosis in which necrosis and apoptosis represent alternate outcomes of the same mitochondrial death pathway.

Further liver diseases are ischemia/reperfusion injury of the liver, death receptor injury and cholestatic liver injury (for a review see Malhi et al., 2006).

Examples of cancers are gastric cancer or hepatic cancer.

It is known that cancer cells can be efficiently eradicated by apoptotic mechanisms. However, disruption of the apoptotic mechanism may cause the survival of cancer cells and the initiation of cell growth. Conventional anticancer therapies exert their effect by inducing apoptosis. However, a disrupted apoptotic pathway often impedes the efficiency of these therapies.

Hematological diseases related to apoptosis can be subdivided into those related to the loss of or escape from apoptosis and those related to excessive apoptosis.

Excessive apoptosis may occur in aplastic anemia, myelodysplastic syndromes (MDS), thalassemia, deficiency of hematopoietic factors such as in renal anemia or cytopenia due to anticancer agents or irradiation. Exemplary diseases and disorders caused by a loss or escape from apoptosis are haematological malignances such as leukaemia, lymphoma or myeloma.

The success of viral infections is often based on strategies to evade apoptosis to permit survival of the virus within the host cell via a range of mechanisms including receptor binding, activation of protein kinase R (PKR) and interaction with p53. Most viruses encode proteins that can inhibit apoptosis. Several viruses encode viral homologs of Bcl-2. These homologs can inhibit pro-apoptotic proteins such as BAX and BAK, which are essential for the activation of apoptosis. Examples of viral Bcl-2 proteins include the Epstein-Barr virus BHRF1 protein and the adenovirus E1B 19K protein. Some viruses express caspase inhibitors that inhibit caspase activity (e.g. the CrmA protein of cowpox viruses). A number of viruses can block the effects of TNF and Fas. For example the M-T2 protein of myxoma viruses can bind TNF preventing it from binding the TNF receptor and inducing a response. Furthermore, many viruses express p53 inhibitors that can bind p53 and inhibit its transcriptional transactivation activity. Consequently p53 cannot induce apoptosis since it cannot induce the expression of pro-apoptotic proteins. The adenovirus E1B-55K protein and the hepatitis B virus HBx protein are examples of viral proteins that can perform such a function.

The progression of the human immunodeficiency virus (HIV) to AIDS is primarily due to the depletion of CD4+ T-helper lymphocytes, which leads to a compromised immune system. One of the mechanisms by which T-helper cells are depleted is apoptosis, which can be the end product of multiple biochemical pathways. In this context the method of identifying an agent capable of regenerating damaged mammalian mitochondria of the present invention could provide potential protective substances to prevent the depletion of T-helper cells by apoptosis in the course of HIV infection.

In another preferred embodiment, the method of the present invention further comprises analysing one or more of the obtained populations with one or more of electron microscopy, immunoblotting, immune electrophoresis, immunoprecipitation, cytofluorometric detection, HPLC, enzymatic activity assays, colocalization studies, detection of inner membrane surface metabolites, NMR, light scattering, atomic force microscopy, two-dimensional gel electrophoresis, optionally in combination with mass spectrometric methods for protein identification or liquid chromatographic methods, optionally in combination with mass spectrometry. Typical references for these methods appear in specialized textbooks like the "Methods in Molecular Biology" or "Methods in Enzymology" series apart from single or review articles (*e.g.* Rabilloud (2002)).

In another preferred embodiment of the method of identifying an agent causing mitochondrial damage in mammalian cells, the test agent is a (cyto)toxic agent.

"(Cyto)toxic agents" as used in connection with the present invention denote agents which are directly toxic to cells. (Cyto)toxic agents causing apoptosis have been described elsewhere in this application. Well known assays to determine cytotoxicity of an agent are *e.g.* the colorimetric MTT, trypan blue, sulforhodamine assay and WST assays and the clonogenic assay.

In a final preferred embodiment of the methods of the present invention relating to the identification of an agent causing mitochondrial damage in mammalian cells or to the identification of an agent capable of regenerating damaged mammalian mitochondria, a library of test agents is screened. Accordingly, the respective methods of the present invention are suitable for the use in high-throughput applications.

A library of test agents can comprise one class of molecules as well as a mixture thereof. Suitable classes of molecules are, inter alia, peptides, nucleic acids, small molecules or aptamers. The general structure of these molecules is well-known in the art.

Peptides are a group of molecules consisting of up to 100 amino acids. Nucleic acids comprise DNA, different types of RNA, such as RNAi, siRNA or shRNA as well as PNAs, a polyamide type of DNA analog. The monomeric units for the corresponding derivatives of adenine, guanine, thymine and cytosine are available commercially (for example from Perceptive Biosystems). PNA is a synthetic DNA-mimic with an amide backbone in place of the sugar-phosphate backbone of DNA or RNA.

Aptamers are oligonucleic acid or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications.

A small molecule according to the present invention can be organic or inorganic and has a molecular weight of up to 2000 Daltons, preferably not more than 1000 Daltons, and most preferably not more than 800 Daltons.

High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to said activity.

The figures show:

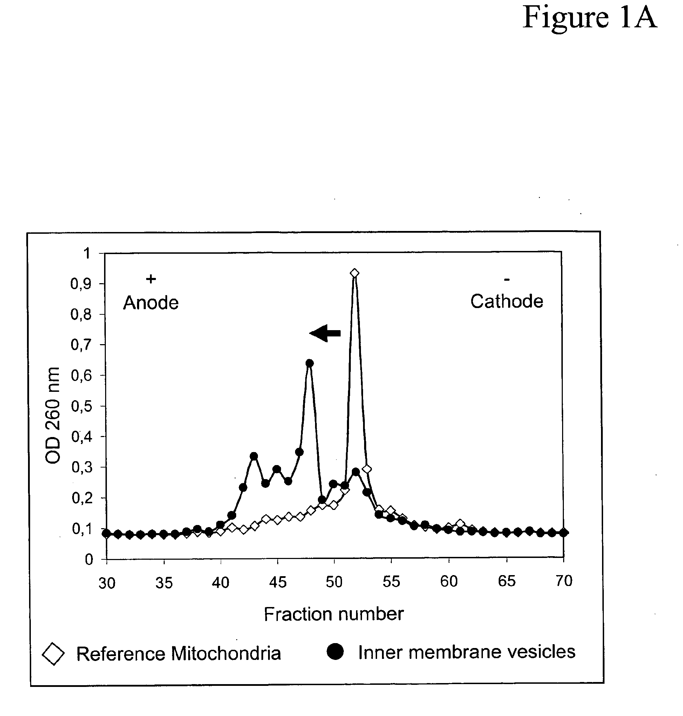

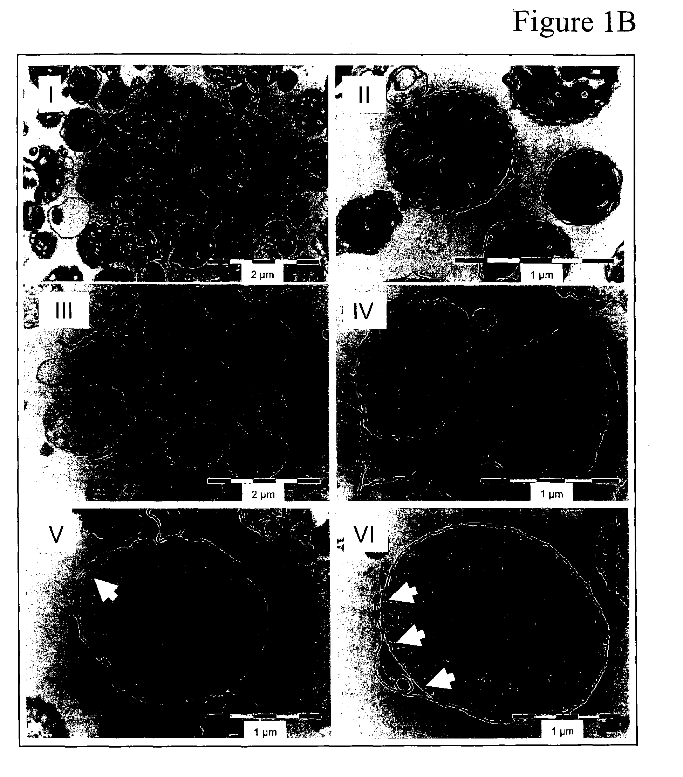

Figure 1: Discrimination of liver mitochondria and inner membrane vesicles by ZE-FFE.

(A) ZE-FFE separation profiles of intact rat/mouse liver mitochondria showed one major peak. Several peaks which deflected more towards the anode (arrow) were observed for IMVs (a representative separation out of 6 experiments with similar results is shown, anode +; cathode -).

(B) Electron micrographs of ZE-FFE purified rat liver mitochondria (I and II) or IMVs (III-VI). Qualitatively more adherent outer membrane was detected in IMVs collected from the more cathodal deflected peaks (arrows in V and VI), as compared to IMVs of the most anodal peak (III and IV).

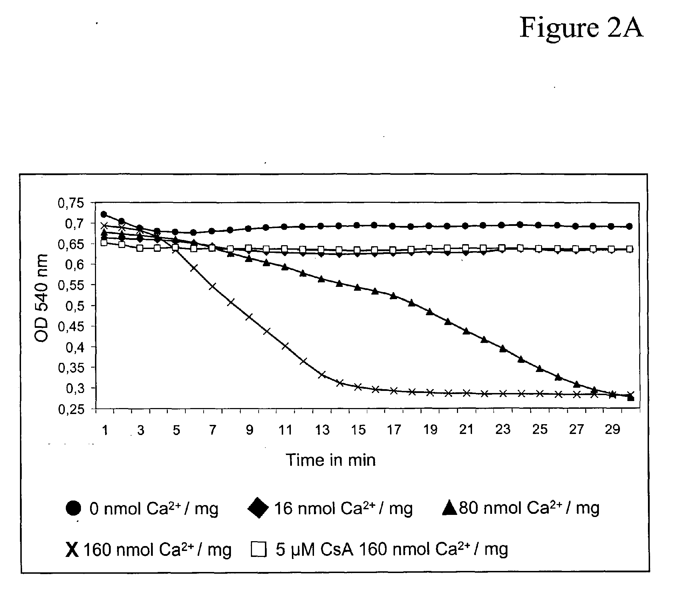

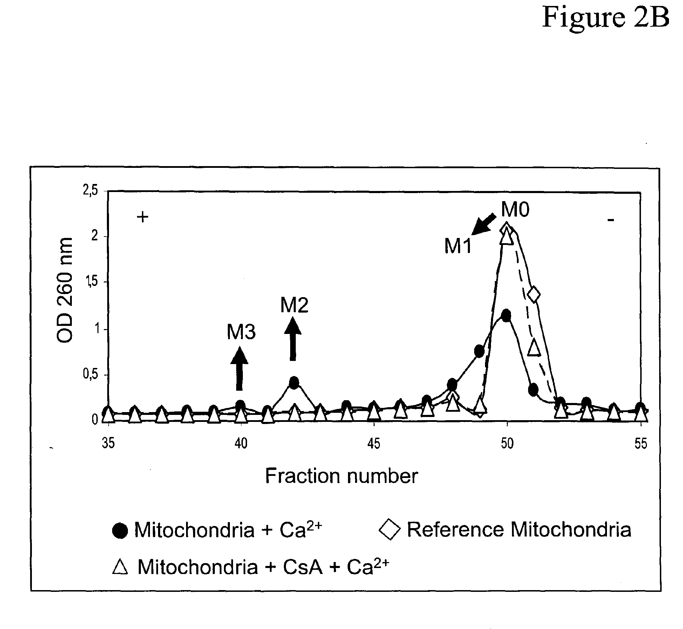

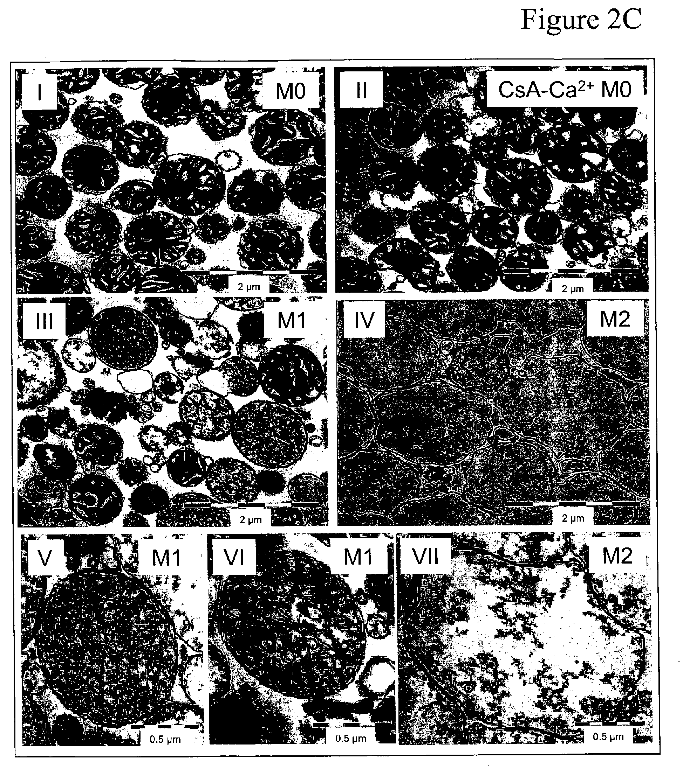

Figure 2: ZE-FFE analysis of rat liver mitochondria undergoing PT.

(A) PT assessment by optical density measurements ("swelling") of mitochondrial suspensions at different $Ca^{2+}$-doses. Swelling occurred with different kinetics and could be inhibited by CsA. Each data curve represents the average of four individual measurements.

(B) PT assessment by ZE-FFE. Untreated reference mitochondria showed one major peak (termed M0). CsA-$Ca^{2+}$-treated mitochondria migrated similar to untreated mitochondria (n=7). In contrast, $Ca^{2+}$-treated mitochondria consistently deflected more towards the anode (n=17). Here, mitochondria were incubated at 25 nmol $Ca^{2+}$/mg for 20 min and subsequent ZE-FFE analysis revealed several peaks (M0-M3, anode +; cathode -, a representative separation out of 9 experiments with similar results is shown)

(C) Electron micrographs of ZE-FFE separated mitochondrial subpopulations. Reference mitochondria and mitochondria treated with CsA-$Ca^{2+}$ collected from the M0 peak (I and II). $Ca^{2+}$-treated mitochondria collected from the M1 peak (III, V and VI) and the M2 peak (IV and VII).

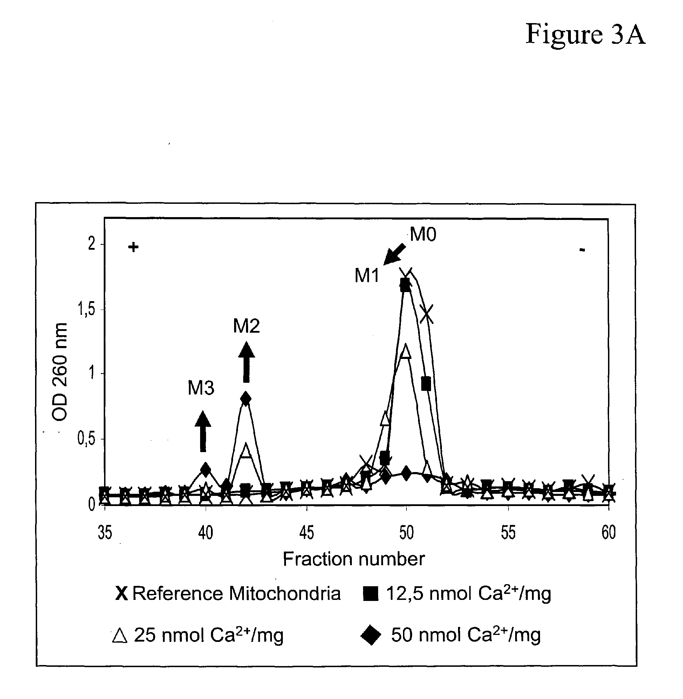

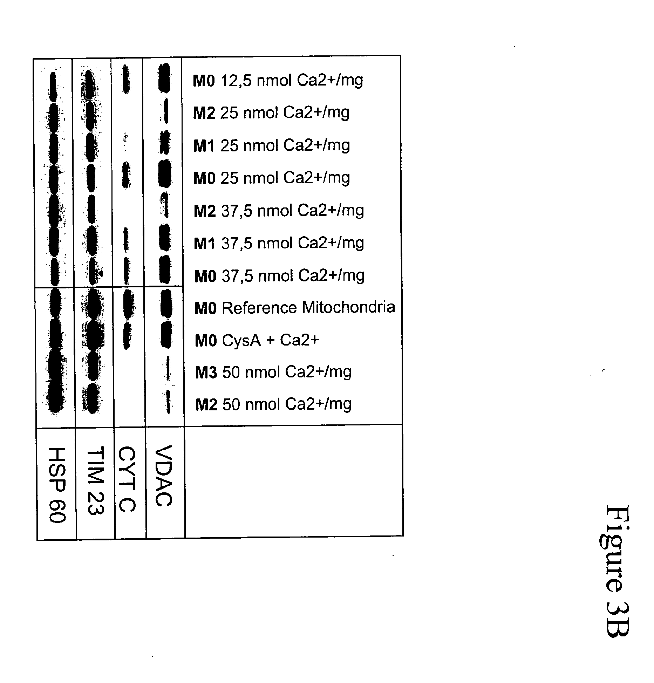

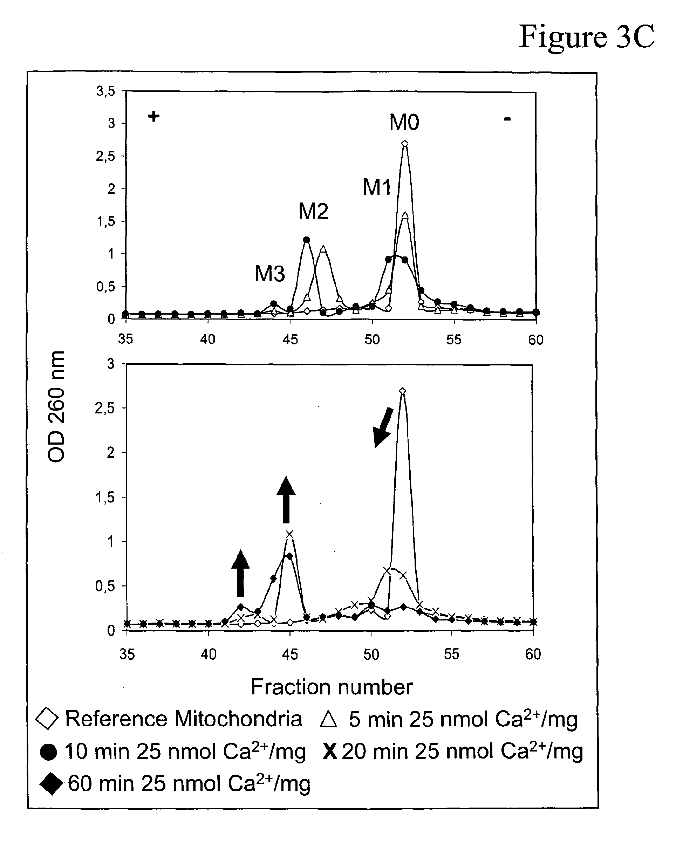

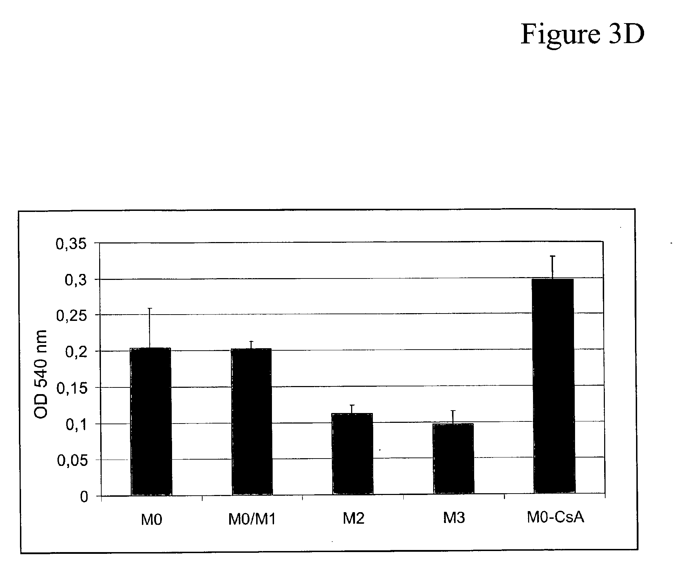

Figure 3: PT causes variable degrees of mitochondrial damage.

(A) ZE-FFE separation profiles of mitochondria treated with different $Ca^{2+}$-doses show quantitative differences in the resulting subpopulations (incubation time 20 min). Increasing $Ca^{2+}$-doses cause a decrease of M0 and M1 and a concomitant increase of M2 and M3 mitochondria (arrows, anode +; cathode -, a representative separation out of 6 experiments with similar results is shown)

(B) Immuno-analysis of ZE-FFE separated mitochondrial subpopulations showed significant differences in their amount of detectable outer membrane VDAC but relatively constant amounts of inner membrane TIM 23 and matrix HSP 60. No Cyt *c* was detected in mitochondria collected from the M2 and M3 mitochondria, while Cyt *c* was present in M1 mitochondria, although to a lower amount as in M0.

(C) ZE-FFE separation profiles of $Ca^{2+}$-treated mitochondria (25 nmol $Ca^{2+}$/mg mitochondria) at different incubation times. Prolonged PT inducing conditions cause a decrease of M0 and M1 and a concomitant increase of M2 and M3 mitochondria (arrows, anode +; cathode -, two independent experiments with at least four time points, each.)

(D) Absorbance measurements at 540 nm of ZE-FFE separated mitochondrial subpopulations normalized to 100 $\mu$g mitochondrial protein of each subpopulation. Data represent average values of three independent mitochondrial preparations and ZE-FFE separations.

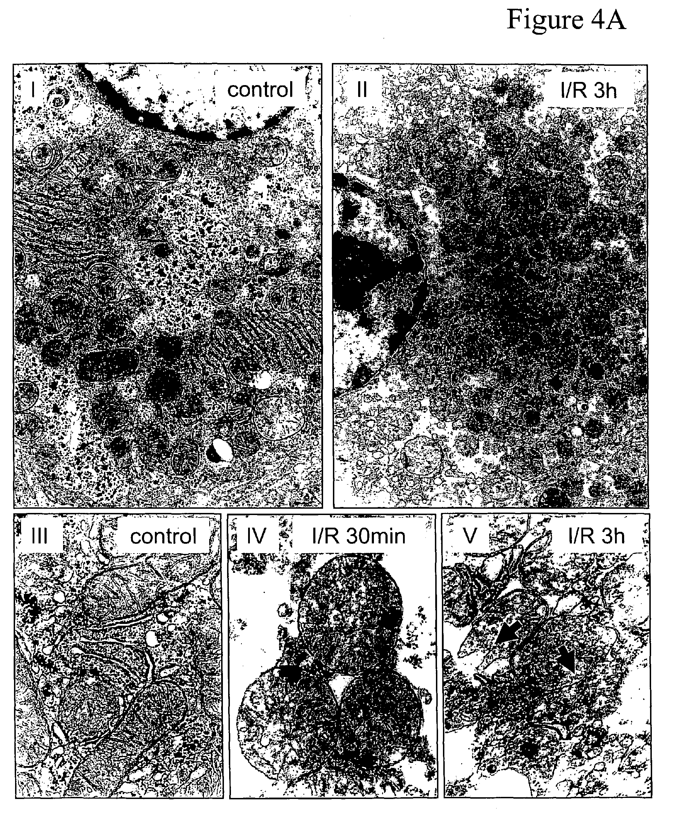

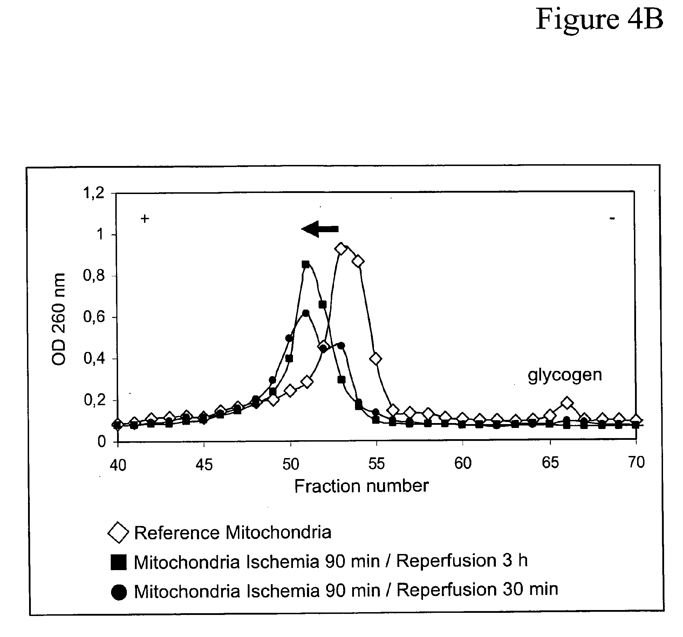

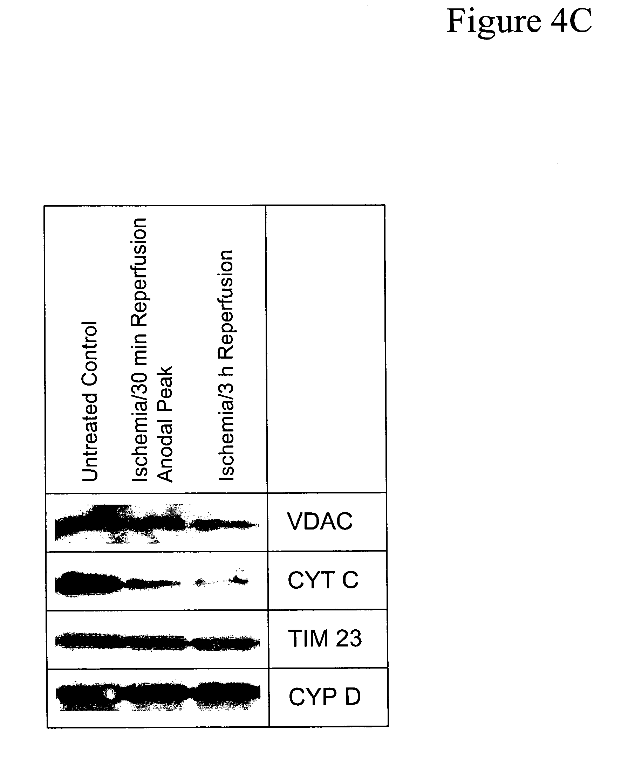

Figure 4: PT-induced MOMP in ischemia/reperfusion.

(A) Electron micrographs of liver tissue demonstrating mitochondrial damage upon I/R. Disintegrating cristae, configurational matrix transitions and outer membrane ruptures (arrows) were noted in mitochondria on electron micrographs of liver tissue subjected to I/R (II, IV and V; reperfusion time was 3 h in II and V and 30 min in IV) in comparison to untreated control tissue (I and III). Magnifications: 21000-fold in I and II and 75000-fold in III-V, respectively.

(B) ZE-FFE separation profile of isolated mitochondria from rat liver treated 90 min with ischemia and 30 min or 3 h reperfusion, respectively, compared to mitochondria isolated from untreated tissues. Control samples demonstrated a major peak and a smaller cathodal peak containing glycogen as identified by positive reaction with Lugol's solution. I/R mitochondria did not contain a glycogen peak. In this example, mitochondria isolated after 30 min reperfusion demonstrated two peaks, one with a deflection similar to control samples and one anodal shifted peak. Organelles isolated after 3 h reperfusion shifted two fractions towards the anode (arrow, a representative separation out of six ZE-FFE experiments with similar results is shown)

(C) Immuno-analysis of collected mitochondria from the ZE-FFE comparison. A slight depletion of outer membrane VDAC and a pronounced depletion of intermembrane space Cyt *c* were found in I/R treated mitochondria in comparison to untreated control mitochondria. Relatively constant amounts of the inner membrane TIM 23 and matrix Cyp D were detected.

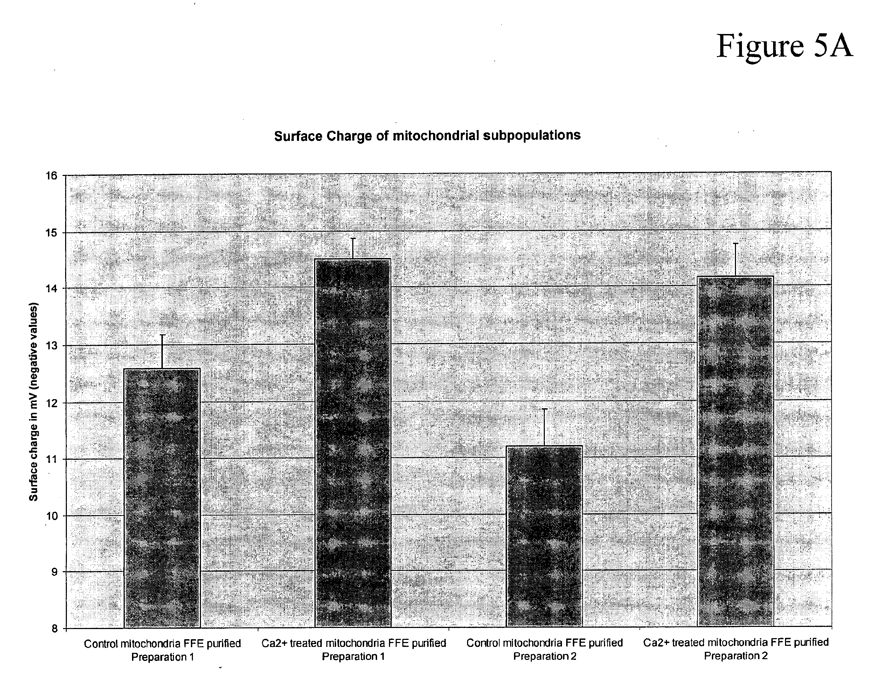

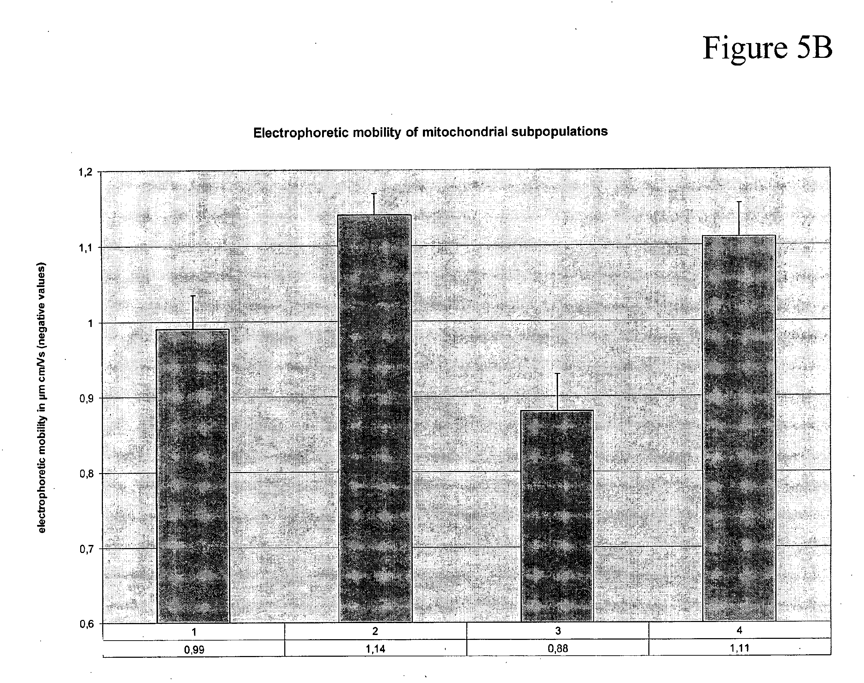

Figure 5: Surface potentials of mitochondrial subpopulations.
Measurement of surface potentials of free flow purified untreated M0control mitochondria *vs.* M2 mitochondria that have undergone a $Ca^{2+}$-induced permeability transition (PT)

(a) Surface charge of mitochondrial subpopulations
(b) Electrophoretic mobility of mitochondrial subpopulations

The examples illustrate the invention.

## Example 1: Methods

*Isolation of rat/mouse liver mitochondria.*

Mitochondria were isolated by differential centrifugation according to standard protocols. Briefly, freshly removed liver tissue was cut into pieces and homogenized with a glass teflon homogenizer in isolation buffer (10 mM triethanolamine (TEA), 10 mM acetic acid (HAc), 280 mM sucrose, 0,2 mM EGTA, pH 7,4 with KOH). Homogenates were cleared from debris and nuclei by two times centrifugation at 750 g (10 min at 4°C) and mitochondria pelleted at 9000 g (10 min at 4°C). Organelles were washed three times (once at 9000 g and two times at 15000 g, 10 min, 4°C) and resuspended in FFE separation buffer (10 mM TEA, 10 mM HAc, 280 mM sucrose, pH 7.4 with KOH).

*Ischemia/reperfusion treatment.*

6 week old male Sprague-Dawley rats (Charles-River-laboratories, Sulzfeld, Germany) were anesthetized by i.p. injection of 0.005 mg/kg Fentanyl (Janssen-Cilag, Neuss, Germany) and 2.0 mg/kg Midazolam (Ratiopharm, Ulm, Germany). Anaesthesia was maintained by 1.5% Isoflurane (Abbott, Wiesbaden, Germany) using a vaporizer with Carbogen (5% $CO_2$/95% $O_2$, Air Liquide, Duesseldorf, Germany), body temperature was maintained with a warming lamp. The abdomen was opened by midline-laparotomy and the portal triad was prepared. The arterial and portal blood flow to the left lateral and median lobe of the liver was interrupted by applying an atraumatic clip, resulting in a 70% liver ischemia. After 90 minutes of ischemia, the blood supply was restored by removal of the clip and the reperfusion period was initiated. Animals were sacrificed after 30 minutes or 3 hours of reperfusion, respectively, by bleeding. Control animals were sacrificed directly after the midline-laparotomy. The livers were rinsed free from blood by perfusing the organs with PBS. Mitochondria were isolated from the left lateral and median lobe of the liver by differential centrifugation as above with the modification that mitochondria were pelleted from the 750 g supernatant at 15000 g (10 min at 4°C) and washed once at 15000 g. Mitochondria were stored in liquid nitrogen according to Fleischer (1979) until further use. For ZE-FFE experiments stored mitochondria were thawed, adapted to FFE separation buffer by washing and subsequently analyzed. For electron-scan microscopy, tissue samples were fixed in 3% glutaraldehyde for 2 hours and then kept in 1% glutaraldehyde until further examination.
All animals received human care in compliance with the "Principles of Laboratory Animal Care". Studies were registered and approved by the government authorities.

*In vitro treatments of isolated rat liver mitochondria.*

IMVs were prepared from mitochondria by osmotic swelling-shrinking as described by Heidrich *et al.* (1970). PT-induced osmotic swelling of freshly isolated rat liver mitochondria suspensions upon $Ca^{2+}$-addition was routinely measured by light scattering at 540 nm in a microplate absorbance reader ($\mu$-Quant™, Bio-Tek, Bad Friedrichshall, Germany) over a period of 30 min at RT. The final assay volume was 200 $\mu$l, containing mitochondria at 0.5 mg/ml in "standard swelling buffer" (10 mM MOPS-Tris pH 7.4, 200 mM sucrose, 5 mM succinate, 1 mM $P_i$, 10 $\mu$M EGTA and 2 $\mu$M rotenone). CsA (5 $\mu$M) was added 5 min before $Ca^{2+}$. Only mitochondrial preparations that showed $Ca^{2+}$-induced PT which could be inhibited by CsA were used for ZE-FFE experiments; coupling of the mitochondrial preparations was routinely checked by standard respiratory measurements.
To study the PT by ZE-FFE 10 mg mitochondria were incubated in 20 ml in "FFE-compatible swelling buffer" (10 mM TEA, 10 mM HAc, 280 mM sucrose, pH 7.4 with KOH, 5 mM succinate, 1 mM $P_i$, 10 $\mu$M EGTA and 2 $\mu$M rotenone) at RT. CsA-inhibited mitochondria were treated with CsA (0.6-4 $\mu$M) for 5 min and $Ca^{2+}$ (25 nmol/mg mitochondria) was subsequently added with 10 min further incubation. $Ca^{2+}$-treated mitochondria were incubated with different $Ca^{2+}$-doses or reaction times as indicated in the text. Treated mitochondria were softly homogenized to ensure proper mixing, pelleted (15.000g, 10min, 4°C), re-suspended and analyzed by ZE-FFE. Untreated reference mitochondria were incubated in FFE-compatible swelling buffer alone, pelleted after 5 min and analyzed by ZE-FFE.

*ZE-FFE analysis of mitochondrial samples*

The mitochondrial samples suspended in FFE separation buffer were analyzed with the aid of a Free-Flow Electrophoresis apparatus (FFE-Weber GmbH, Planegg, Germany) with the following conditions: (i) circuit electrolyte solution was 100 mM HAc, 100 mM TEA adjusted to pH 7.4 with KOH; electrolyte stabilizing solution was 100 mM HAc, 100 mM TEA, 0.28 M sucrose, pH 7.4, FFE separation buffer was 10 mM TEA, 10 mM HAc, 280 mM sucrose, pH 7.4; (ii) all buffers were cooled on ice during separation to avoid thermal gradients; (iii) mitochondrial samples were applied to ZE-FFE with a sample flow rate of 1-2 ml/h; (iv) electrophoresis was performed in horizontal mode at 5°C with a flow rate of 330 ml/h and a voltage of 750 V; (v) fractions were collected in 96-well plates and the distribution of resolved particles was monitored at a wavelength of 260 nm with a $\mu$-Quant™-microplate reader (Bio-Tek instruments, Bad Friedrichshall, Germany); (vi) ZE-FFE separated mitochondrial fractions were concentrated by centrifugation at 4°C (10 min at 16000 g);(vii) in order to ensure constant separation conditions during the ZE-FFE analysis of different mitochondrial subpopulations, control experiments and routine tests of the ZE-FFE apparatus were conducted as described elsewhere (Zischka et al., 2006); (viii) comparisons of mitochondrial subpopulations, *e.g.*, dose dependent $Ca^{2+}$-treated mitochondria, were done consecutively on the same day, under identical conditions regarding voltage, buffer composition, buffer and sample velocity and temperature. From each sample, several separation profiles were recorded to ensure time stability and exclude drifts in separation conditions.

*Electron microscopy*

ZE-FFE-separated mitochondrial fractions were immediately pelleted, fixed in 3% glutaraldehyde, post fixed with 1% osmium tetroxide, dehydrated with ethanol and embedded in Epon. Ultra-thin sections were negative stained with uranyl acetate and lead citrate and then analyzed on a Zeiss EM 10 CR electron microscope. Glutaraldehyde fixed liver tissues were treated similar but dehydrated with acetone and analyzed on a Philips EM 420 electron microscope.

*Miscellaneous*

Quantification of mitochondria was done by the Bradford assay. Immunoblotting of ZE-FFE-separated mitochondrial protein extracts was carried out according to Towbin *et al.* (1979), using polyclonal antisera against VDAC (Acris) and monoclonal antibodies against Cyt *c*, TIM23, HSP60 (BD) and Cyp D (Acris).

**Example 2: Discrimination of intact rat liver mitochondria and inner membrane vesicles by ZE-FFE.**

Mitochondria have an "envelope-like" outer membrane and a structured inner membrane (Mannella, 2006). Permeabilities dramatically differ between these membranes, the outer membrane being freely permeable to low molecular weight solutes like sucrose, and the inner membrane being sucrose-impermeable. Incubation of purified mitochondria in low-osmolar or high-osmolar sucrose solutions leads to matrix water uptake and osmotic swelling or shrinkage, respectively. Such osmotic treatments rupture the mitochondrial outer membranes and generate inner membrane-matrix vesicles (IMVs) that can be electrophoretically distinguished from untreated mitochondria (Heidrich et al., 1970). Due to the more negative surface charge of the inner compared to the outer mitochondrial membrane of rat liver mitochondria (Heidrich et al., 1970), a stronger anodal deflection of the IMVs occurred in ZE-FFE (Fig 1A). Interestingly, we observed several IMV-peaks in the ZE-FFE separation profile (Fig 1A). Electron microscopy analysis of the mitochondrial structures collected from these peaks revealed a qualitative difference in the amount of residual outer membrane adherent to the inner membrane. Hardly any outer membrane was observed in IMVs collected from the most anodal peak; however, IMVs with ever more abundant contaminations of the outer membrane were detected from the more cathodal peaks (Fig. 1B, arrows in V and VI). It thus appears that the relative abundance of the inner and outer membranes determine the deflection in ZE-FFE, suggesting that the relative content of residual outer mitochondrial membrane on damaged mitochondrial structures can be monitored by ZE-FFE.

**Example 3: Preparative analysis of mitochondria undergoing PT by ZE-FFE.**

Isolated mitochondria undergo PT *in vitro*, upon the addition of $Ca^{2+}$ in the presence of phosphate, and this process can be inhibited by CsA (Crompton et al., 1988). PT is typically monitored as a decrease in light scattering caused by mitochondrial matrix swelling (Beatrice et al., 1980) (Fig 2A). Due to its limited extensibility, the outer membrane ruptures upon matrix swelling (Van der Heiden et al., 1997). We reasoned that (i) the PT-induced break-up of the mitochondrial outer membrane should expose parts of the inner membrane and that (ii) due to the more negative charged surface of the mitochondrial inner membrane (Fig 1 A), a stronger deflection of PT-induced mitochondria in comparison to intact mitochondria towards the anode should occur in ZE-FFE.

Isolated rat liver mitochondria were energized with succinate, challenged for 20 min at RT with $Ca^{2+}$ (25 nmol/mg mitochondria) and subsequently analyzed by ZE-FFE (Fig 2B). Energized mitochondria from the same preparation but without $Ca^{2+}$-addition ("reference mitochondria") were chosen to assess and compare the ZE-FFE deflection of undamaged mitochondria. Energized mitochondria that were pre-treated with CsA before $Ca^{2+}$ addition ("CsA-inhibited mitochondria") were used as PT-inhibited control.

Reference mitochondria eluted in one major peak that we designated M0 (Fig 2B). CsA-inhibited mitochondria were also eluted in one peak which deflected similar to reference mitochondria, although sometimes a slight anodal shift was noticed (Fig 2B). The M0 mitochondria had intact outer membranes, regular cristae structures and an electron dense matrix staining was observed (Fig 2C, I and II). In contrast, $Ca^{2+}$-treated mitochondria, separated into several peaks (and shoulder peaks), which were labelled M0, M1, M2 and M3 from the cathode to the anode, respectively (Fig 2B). Depending on the experiment, in ZE-FFE separation profiles of $Ca^{2+}$-treated mitochondria the most cathodal peak, shifted one fraction more towards the anode (and was designated M1) as compared to the M0 peak of reference mitochondria (not shown). Alternatively, and in most experiments, compared to the M0 peak of reference mitochondria, a peak broadening of around one to two fractions towards the anode was noted (Fig 2B). Mitochondria collected from the anodal shoulder of this broadened peak were designated M1 and mitochondria from the cathodal shoulder which deflected similar to the reference mitochondria were designated M0. Electron microscopy revealed a morphologically heterogeneous mixture of mitochondria collected from M1 peaks (Fig 2C, III), comprising mitochondria resembling intact reference mitochondria as well as a large portion of mitochondria which possessed dilated matrices and disorganised cristae (Fig 2C, V and VI). M1 mitochondria were still surrounded by an outer membrane which was, however, damaged to different extents (Fig 2C, III and below). In comparison to M1, M2 mitochondria were homogeneous vesicles with IMV morphology (Fig 2C, IV and VII) and little outer membrane was detected in M2 mitochondria (Fig 2C). These data show that $Ca^{2+}$-induced PT causes anodal deflection of mitochondria in ZE-FFE. Mitochondria which have undergone a full-blown PT-induced MOMP (*i.e.* M2) can clearly be separated from other mitochondria by ZE-FFE.

**Example 4: $Ca^{2+}$-induced PT causes variable degrees of mitochondrial damage in a dose- and time-dependent fashion.**

In "classical" swelling measurements increasing $Ca^{2+}$-doses are added to mitochondrial suspensions while the absorbance or diffraction of light (at 540 nm) is monitored. Above a certain threshold of $Ca^{2+}$, PT-associated swelling occurs as assessed by a decrease in the absorbance (Fig 2A). Depending on the $Ca^{2+}$-dose, different kinetics of swelling are observed. The higher the $Ca^{2+}$ dose, the more rapid is the reduction of the OD540 (Fig 2A). However, this methodology monitors the response of the entire mitochondrial subpopulation towards $Ca^{2+}$. As a consequence, we decided to employ ZE-FFE to monitor the possible generation of heterogeneously damaged mitochondria upon PT induction by different $Ca^{2+}$-doses and at different time points.

Energized mitochondria were incubated for 20 min at RT with $Ca^{2+}$-doses ranging from 12.5 nmol $Ca^{2+}$/mg mitochondria to 50 nmol $Ca^{2+}$/mg mitochondria and subsequently analyzed by ZE-FFE (Fig 3A). At a dose of 12.5 nmol $Ca^{2+}$/mg mitochondria, one major peak was detectable in the ZE-FFE separation profile (Fig 3A), which was almost indistinguishable from that obtained with untreated reference mitochondria (Fig 3A). At 25 nmol $Ca^{2+}$/mg, a reduction of this major M0 peak together with a broadening towards the anode (M1) as well as the emergence of the more anodal M2 peak was noted (Fig 3A). Further depletion of the M0/M1 peak was found at 37.5 nmol $Ca^{2+}$/mg (not shown). At 50

nmol $Ca^{2+}$/mg the M0/M1 peak was hardly detectable, coinciding with an increase of the M2 peak and the emergence of a further anodal peak (M3) (Fig 3A). It should be noted that the sensitivities of mitochondria towards $Ca^{2+}$-induced PT differed somewhat between the individual preparations. The given $Ca^{2+}$ doses should thus not be misconceived as absolute values but rather as the doses and sensitivities typically observed in the great majority of these experiments.

Due to the preparative character of the ZE-FFE analysis, the different subpopulations can be recovered and hence are amenable to downstream analyses like electron microscopy (Fig 2C) or proteomics. In fact, subsequent immuno-blotting of protein extracts of the organelles collected from these peaks (Fig 3B) demonstrated significant differences in the amount of outer membrane (VDAC) and intermembrane space (Cyt c) proteins but showed relatively constant amounts of inner membrane (TIM 23) and matrix proteins (HSP 60). With respect to the reference mitochondria (M0), the more anodal the sub-fraction was collected the more substantial was the depletion in VDAC (Fig. 3B). No Cyt c was detected in mitochondria collected from the M3 and M2 mitochondria, while Cyt c was still present although at reduced levels in M1 mitochondria as compared to M0 (Fig 3B).

To study the time-dependent alteration of mitochondrial subpopulations upon PT-induction by ZE-FFE, energized mitochondria were incubated with 25 nmol $Ca^{2+}$/mg mitochondria for 5 to 60 min and subsequently analyzed (Fig 3C). A decrease of the major M0 peak and the emergence of the M2 peak were already noted in ZE-FFE separation profiles of mitochondria after 5 min PT induction (Fig 3C, upper panel). At 10 min the M0/M1 peak broadening occurred and the M2 and M3 peaks became clearly detectable (Fig 3C, upper panel). Similarly, at 20 min a further depletion of M0/M1 mitochondria was observed (Fig 3C, lower panel). After 40 min (not shown) and 60 min incubation, M0 disappeared, leaving a small residual peak as well as prominent M2 and M3 peaks (Fig 3C, lower panel).

In order to assess how the different separated mitochondrial subpopulation(s) contribute to the changes in "classical" swelling assays, we determined their spectrometric properties at OD 540 nm (Fig 3D). Similar absorbance values were found for reference mitochondria collected from the M0 peak and from $Ca^{2+}$-treated mitochondria collected from the M0/M1 peak (Fig 3D). In contrast, $Ca^{2+}$-treated mitochondria collected from the M2 and M3 peaks showed significantly lower absorbance values (Fig 3D). It thus occurs that the absorbance decrease in "classical" swelling assays can be related to the emergence of M2 and M3 mitochondria in ZE-FFE. Consequently, a fast decrease in absorbance in swelling measurements translates into a major/fast transition from M0/M1 to M2/M3 mitochondria in ZE-FFE. Interestingly, ZE-FFE purified CsA-inhibited mitochondria gave a slightly higher OD 540 nm value compared to reference mitochondria (Fig 3D), which is in agreement with the frequent observation of an absorbance increase of CsA-treated mitochondrial suspensions upon Ca2+ addition.

Taken together, these data indicate that ZE-FFE is suitable for the quantitative and qualitative assessment of mitochondrial damage, as it is inflicted by $Ca^{2+}$-induced PT in a dose- and time-dependent fashion.

**Example 5: PT-induced MOMP in ischemia/reperfusion analyzed by ZE-FFE.**

PT has been considered as an *in vitro* artefact for a long time (Bernardi et al., 2006), and many unsolved questions surround the role of the mitochondrial PT *in vivo*. It is generally accepted that mitochondrial membrane permeabilization represents a committing step in cell death scenarios (Green and Kroemer, 2004). It is a matter of controversy, however, whether PT is a cause or a consequence of cell death (Kinally and Antonsson, 2007), under which conditions and to which extent PT occurs *in vivo* (Bernardi et al., 2006), and which threshold of PT is associated with cell death in distinct cell types (Rodriguez-Enriquez et al., 2004). However, there is some consensus that PT occurs and contributes to massive cell death during the reperfusion phase of tissues which have undergone a long period of ischemia (I/R), *e.g*. due to surgery, thrombosis or stroke (Bernardi et al., 2006; Halestrap, 2006; Saris and Eriksson, 1995). Ischemia leads to mitochondrial de-energization, ATP depletion, lactic acid accumulation and consequent intracellular pH drop. Consequently cells are loaded with $Ca^{2+}$ due to the action of the $Na^+/H^+$ and $Na^+/Ca^{2+}$-antiporters. Upon reperfusion, pH normalizes, mitochondria re-energize and take up $Ca^{2+}$, setting the conditions for PT to occur (Halestrap, 2006).

Indeed, and in agreement with earlier reports (Saris and Eriksson, 1995), pronounced mitochondrial alterations were observed in electron micrographs from fixed liver cells upon I/R (Fig 4A). In contrast to the ellipsoid-shaped, tubular cristae containing mitochondria from untreated liver tissues (Fig 4A, I and III), mitochondria in I/R tissues were markedly rounded, appeared swollen and had lost their typical cristae organization (Fig 4A, II, IV and V). Moreover, mitochondria presented partial outer membrane ruptures and losses (Fig 4A, V). In contrast, I/R did not induce the appearance of vesicles with IMV morphology that would resemble M2 mitochondria (Fig 2C, VII), suggesting that a full-blown PT did not occur under these conditions. Rather, I/R-damaged mitochondria morphologically resembled M1 mitochondria (compare Fig 4C, II and IV with Fig 2C, III, V and VI).

Next, we compared I/R mitochondria and untreated control mitochondria by ZE-FFE (Fig 4B). The ZE-FFE separation profiles of control samples yielded a major peak containing mitochondria and a smaller cathodal glycogen-containing peak. This glycogen peak was absent from I/R mitochondria, presumably as a result of glycogen catabolism. A quantitative anodal shift was seen for I/R mitochondria as compared to controls (Fig 4B), and this effect increased upon protracted reperfusion (compare profiles for 30 min and 3 h in Fig. 4B).

Immuno-blotting analyses of ZE-FFE-purified mitochondrial fractions demonstrated a slight depletion of outer membrane VDAC but a substantial depletion of Cyt *c* in I/R mitochondria versus untreated control mitochondria (Fig 4C). In contrast, little variations were observed for the inner membrane protein TIM 23 and the matrix protein Cyp D (Fig 4C).

Thus, electron microscopy, ZE-FFE and the subsequent immuno-blotting analysis consistently demonstrated mitochondrial damage upon I/R. It appeared, however, that in contrast to the observed mitochondrial alterations from *in vitro* experiments, I/R-induced a less pronounced mitochondrial damage with respect to outer membrane ruptures and losses, suggesting that severe loss of the outer membrane upon I/R leading to the formation of IMV-like structures does not occur *in vivo.*

**Example 6: Confirmation of different surface potentials by determining the zeta potential.**

In order to quantitatively assess the different surface potentials of untreated control mitochondria *vs.* mitochondria that have undergone a $Ca^{2+}$-induced permeability transition (PT) we had these organelles subjected to an alternative electrophoretic apparatus, the Zetasizer Nano ZS™. Free flow purified M0 and M2 mitochondria from two independent experiments were measured (Table 1). As a result, M2 (*i.e.* $Ca^{2+}$-induced PT) mitochondria in comparison to M0 control mitochondria, gave significantly higher negative values in surface charge (Fig. 5A) and electrophoretic mobility (Fig. 5B). These data are in agreement with the separation results of our free flow experiments, which showed that M2 mitochondria deflect more to the positively charged anode in comparison to M0 control mitochondria. Thus, both electrophoretic methods are sensitive to the altered surface potential of PT-damaged mitochondria in comparison to control organelles. On one hand this result is to be expected, since both methods are sensitive to the electrophoretic mobility of the investigated particles, on the other hand, however, this result shows that it is the mitochondrial damage/difference which is detected independently of the method used to assess it. Consequently both methods may be employed to detect mitochondrial damage, thus enabling the use of the appropriate technique depending on the experimental circumstances. The Zetasizer appears to have clear advantages regarding low amounts of analysis material (*e.g.* originating from biopsies or cell culture) and low analysis time. The electrophoretic free flow approach is a preparative method by which the separated mitochondria can be collected and subsequently analyzed by biochemical or functional methods. Furthermore several subpopulations emerging from damaging conditions may be separated by this latter approach, thus resulting in a more detailed analytical picture.

**Summary and conclusion**

Irreversible outer mitochondrial membrane damage turns the cellular switch towards death and several approaches aim to monitor this "point of no return". Here, we present a novel method for monitoring and characterizing cell death-associated mitochondrial alterations by electrophoretic methods. Due to the more negative charged surface of the inner membrane of rat liver mitochondria, which is exposed after the rupture or loss of the outer membrane, a stronger deflection of OM-ruptured mitochondria towards the anode occurs in comparison to intact mitochondria for example in ZE-FFE. This approach provides an analytical tool and, more importantly, a preparative method for the purification of mitochondria from higher eukaryotes that have undergone distinct level of damage *in vitro* or *in vivo.*

Table 1: Samples 1 to 4 measured on July 12 2007

Samples 1 to 4 were measured in their original medium
measurements conducted on the Zetasizer Nano ZS device with the standard sample cell DTS1060

| Record | Type | Sample Name | Measurement Date and Time | T °C | ZP mV | Mob μmcm/Vs | Cond mS/cm | Current mA | Voltage V | Quality | Zeta Runs |
|---|---|---|---|---|---|---|---|---|---|---|---|
| 1 | Zeta | sample 1 FIII 02.07.07 control 1 | Friday, July 13 2007 15:05:10 | 25 | -12,4 | -0,9687 | 0,654 | 0,253 | 30 | 1,76 | 12 |
| 2 | Zeta | sample 1 FIII 02.07.07 control 2 | Friday, July 13 2007 15:06:11 | 25 | -11,5 | -0,9036 | 0,655 | 0,253 | 30 | 1,46 | 12 |
| 3 | Zeta | sample 1 FIII 02.07.07 control 3 | Friday, July 13 2007 15:07:26 | 25 | -13,2 | -1,031 | 0,656 | 0,253 | 30 | 1,59 | 12 |
| 4 | Zeta | sample 1 FIII 02.07.07 control 4 | Friday, July 13 2007 15:08:41 | 25 | -12,4 | -0,9731 | 0,656 | 0,253 | 30 | 1,21 | 13 |
| 5 | Zeta | sample 1 FIII 02.07.07 control 5 | Friday, July 13 2007 15:10:02 | 25 | -12 | -0,9437 | 0,657 | 0,253 | 30 | 1,65 | 12 |
| 6 | Zeta | sample 1 FIII 02.07.07 control_ 2 1 | Friday, July 13 2007 15:12:45 | 25 | -13,5 | -1,062 | 0,658 | 0,34 | 40 | 2,9 | 12 |
| 7 | Zeta | sample 1 FIII 02.07.07 control_ 2 2 | Friday, July 13 2007 15:13:42 | 25 | -13 | -1,023 | 0,659 | 0,34 | 40 | 1,64 | 12 |
| 8 | Zeta | sample 1 FIII 02.07.07 control_ 2 3 | Friday, July 13 2007 15:15:01 | 25 | -12,6 | -0,9898 | 0,659 | 0,34 | 40 | 1,75 | 12 |
| 9 | Zeta | sample 1 FIII 02.07.07 control_ 2 4 | Friday, July 13 2007 15:16:22 | 25 | -12,2 | -0,9574 | 0,66 | 0,34 | 40 | 1,59 | 12 |
| 10 | Zeta | sample 1 FIII 02.07.07 control 2 5 | Friday, July 13 2007 15:17:41 | 25 | -13,1 | -1,029 | 0,659 | 0,34 | 40 | 1,97 | 12 |
| | | **mean value** | | | **-12,59** | **-0,98813** | 0,6573 | 0,2965 | | | |
| | | **STABW N** | | | **0,582** | **0,046** | 0,002 | 0,044 | | | |
| 11 | Zeta | sample 2 FI 02.07.07 vesicle 1 | Friday, July 13 2007 15:20:00 | 25 | -14,8 | -1,159 | 0,661 | 0,341 | 40 | 1,96 | 12 |
| 12 | Zeta | sample 2 FI 02.07.07 vesicle 2 | Friday, July 13 2007 15:21:01 | 25 | -14,4 | -1,13 | 0,661 | 0,341 | 40 | 2,67 | 12 |

Samples 1 to 4 were measured in their original medium measurements conducted on the Zetasizer Nano ZS device with the standard sample cell DTS1060

| Record | Type | Sample Name | Measurement Date and Time | T °C | ZP mV | Mob μmcm/Vs | Cond mS/cm | Current mA | Voltage V | Quality | Zeta Runs |
|---|---|---|---|---|---|---|---|---|---|---|---|
| 13 | Zeta | sample 2 FI 02.07.07 vesicle 3 | Friday, July 13 2007 15:22:19 | 25 | -13,9 | -1,093 | 0,661 | 0,341 | 40 | 2,41 | 12 |
| 14 | Zeta | sample 2 FI 02.07.07 vesicle 4 | Friday, July 13 2007 15:23:37 | 25 | -14,9 | -1,172 | 0,661 | 0,341 | 40 | 2,1 | 12 |
| 15 | Zeta | sample 2 FI 02.07.07 vesicle 5 | Friday, July 13 2007 15:24:54 | 25 | -14,5 | -1,135 | 0,661 | 0,341 | 40 | 1,68 | 12 |
| 16 | Zeta | sample 2 FI 02.07.07 vesicle_ 2 1 | Friday, July 13 2007 15:27:50 | 25 | -14,6 | -1,145 | 0,663 | 0,342 | 40 | 2,71 | 12 |
| 17 | Zeta | sample 2 FI 02.07.07 vesicle_ 2 2 | Friday, July 13 2007 15:28:50 | 25 | -13,8 | -1,082 | 0,664 | 0,342 | 40 | 2,82 | 12 |
| 18 | Zeta | sample 2 FI 02.07.07 vesicle_ 2 3 | Friday, July 13 2007 15:30:07 | 25 | -14,7 | -1,149 | 0,666 | 0,343 | 40 | 1,96 | 12 |
| 19 | Zeta | sample 2 FI 02.07.07 vesicle_ 2 4 | Friday, July 13 2007 15:31:26 | 25 | -14,9 | -1,166 | 0,666 | 0,343 | 40 | 2,08 | 12 |
| 20 | Zeta | sample 2 FI 02.07.07 vesicle_ 2 5 | Friday, July 13 2007 15:32:43 | 25 | -14,5 | -1,139 | 0,666 | 0,343 | 40 | 1,93 | 12 |
| | | **mean value** | | | **-14,5** | **-1,137** | 0,663 | 0,3418 | | | |
| | | **STABW N** | | | **0,363** | **0,028** | 0,002 | 0,001 | | | |
| 21 | Zeta | sample 3 FIII 06.07.07 control 1 | Friday, July 13 2007 15:36:23 | 25 | -11,5 | -0,8993 | 0,658 | 0,338 | 40 | 2,02 | 12 |
| 22 | Zeta | sample 3 FIII 06.07.07 control 2 | Friday, July 13 2007 15:37:22 | 25 | -11,6 | -0,9106 | 0,653 | 0,336 | 40 | 1,89 | 12 |
| 23 | Zeta | sample 3 FIII 06.07.07 control 3 | Friday, July 13 2007 15:38:39 | 25 | -11,9 | -0,9319 | 0,651 | 0,335 | 40 | 2,74 | 12 |
| 24 | Zeta | sample 3 FIII 06.07.07 control 4 | Friday, July 13 2007 15:40:01 | 25 | -10,9 | -0,8551 | 0,65 | 0,335 | 40 | 1,69 | 12 |

Samples 1 to 4 were measured in their original medium measurements conducted on the Zetasizer Nano ZS device with the standard sample cell DTS1060

| Record | Type | Sample Name | Measurement Date and Time | T °C | ZP mV | Mob μmcm/Vs | Cond mS/cm | Current mA | Voltage V | Quality | Zeta Runs |
|---|---|---|---|---|---|---|---|---|---|---|---|
| 25 | Zeta | sample 3 FIII 06.07.07 control 5 | Friday, July 13 2007 15:41:21 | 25 | -11,3 | -0,8882 | 0,649 | 0,335 | 40 | 2,2 | 12 |
| 26 | Zeta | sample 3 FIII 06.07.07 control_ 2 1 | Friday, July 13 2007 15:47:13 | 25 | -11,5 | -0,8984 | 0,649 | 0,335 | 40 | 1,69 | 12 |
| 27 | Zeta | sample 3 FIII 06.07.07 control_ 2 2 | Friday, July 13 2007 15:48:13 | 25 | -11,2 | -0,876 | 0,65 | 0,335 | 40 | 1,9 | 12 |
| 28 | Zeta | sample 3 FIII 06.07.07 control_ 2 3 | Friday, July 13 2007 15:49:33 | 25 | -11 | -0,8634 | 0,65 | 0,335 | 40 | 1,04 | 15 |
| 29 | Zeta | sample 3 FIII 06.07.07 control_ 2 4 | Friday, July 13 2007 15:51:00 | 25 | -10,5 | -0,8262 | 0,65 | 0,335 | 40 | 1,35 | 12 |
| 30 | Zeta | sample 3 FIII 06.07.07 control_ 2 5 | Friday, July 13 2007 15:52:19 | 25 | -9,53 | -0,7468 | 0,651 | 0,335 | 40 | 1,82 | 12 |
| 31 | Zeta | sample 3 FIII 06.07.07 control_ 3 1 | Friday, July 13 2007 15:55:05 | 25 | -12,1 | -0,9457 | 0,652 | 0,336 | 40 | 1,93 | 12 |
| 32 | Zeta | sample 3 FIII 06.07.07 control_ 3 2 | Friday, July 13 2007 15:56:06 | 25 | -11,7 | -0,916 | 0,652 | 0,336 | 40 | 1,44 | 12 |
| 33 | Zeta | sample 3 FIII 06.07.07 control_ 3 3 | Friday, July 13 2007 15:57:25 | 25 | -11,6 | -0,9064 | 0,652 | 0,336 | 40 | 2,02 | 12 |
| 34 | Zeta | sample 3 FIII 06.07.07 control_ 3 4 | Friday, July 13 2007 15:58:44 | 25 | -11,4 | -0,8937 | 0,652 | 0,336 | 40 | 2,24 | 12 |
| 35 | Zeta | sample 3 FIII 06.07.07 control_ 3 5 | Friday, July 13 2007 16:00:04 | 25 | -10,3 | -0,8109 | 0,652 | 0,336 | 40 | 1,19 | 12 |

Samples 1 to 4 were measured in their original medium
measurements conducted on the Zetasizer Nano ZS device with the standard sample cell DTS1060

| Record | Type | Sample Name | Measurement Date and Time | T °C | ZP mV | Mob µmcm/Vs | Cond mS/cm | Current mA | Voltage V | Quality | Zeta Runs |
|---|---|---|---|---|---|---|---|---|---|---|---|
| | | **mean value** | | | **-11,202** | **-0,8779** | 0,6514 | 0,3356 | | | |
| | | **STABW N** | | | **0,6490** | **0,0498** | 0,0021 | 0,0008 | | | |
| 36 | Zeta | sample 4 FI 06.07.07 vesicle 1 | Friday, July 13 2007 16:02:07 | 25 | -14,4 | -1,125 | 0,645 | 0,332 | 40 | 1,6 | 12 |
| 37 | Zeta | sample 4 FI 06.07.07 vesicle 2 | Friday, July 13 2007 16:03:08 | 25 | -13,8 | -1,085 | 0,646 | 0,333 | 40 | 2,39 | 12 |
| 38 | Zeta | sample 4 FI 06.07.07 vesicle 3 | Friday, July 13 2007 16:04:27 | 25 | -13,6 | -1,063 | 0,647 | 0,334 | 40 | 2,33 | 12 |
| 39 | Zeta | sample 4 FI 06.07.07 vesicle 4 | Friday, July 13 2007 16:05:45 | 25 | -13,8 | -1,079 | 0,646 | 0,334 | 40 | 1,77 | 12 |
| 40 | Zeta | sample 4 FI 06.07.07 vesicle 5 | Friday, July 13 2007 16:07:03 | 25 | -13,9 | -1,09 | 0,647 | 0,334 | 40 | 1,75 | 12 |
| 41 | Zeta | sample 4 FI 06.07.07 vesicle_ 2 1 | Friday, July 13 2007 16:23:31 | 25 | -15,3 | -1,199 | 0,65 | 0,336 | 40 | 2,24 | 12 |
| 42 | Zeta | sample 4 FI 06.07.07 vesicle_ 2 2 | Friday, July 13 2007 16:24:34 | 25 | -14,3 | -1,123 | 0,65 | 0,336 | 40 | 1,7 | 12 |
| 43 | Zeta | sample 4 FI 06.07.07 vesicle_ 2 3 | Friday, July 13 2007 16:25:53 | 25 | -13,3 | -1,041 | 0,651 | 0,336 | 40 | 2,06 | 12 |
| 44 | Zeta | sample 4 FI 06.07.07 vesicle_ 2 4 | Friday, July 13 2007 16:27:12 | 25 | -13,4 | -1,05 | 0,652 | 0,338 | 40 | 2,11 | 12 |
| 45 | Zeta | sample 4 FI 06.07.07 vesicle_ 2 5 | Friday, July 13 2007 16:28:31 | 25 | -14,8 | -1,159 | 0,651 | 0,337 | 40 | 2,37 | 12 |
| 46 | Zeta | sample 4 FI 06.07.07 vesicle_ 3 1 | Friday, July 13 2007 17:45:32 | 25 | -13,5 | -1,06 | 0,653 | 0,337 | 40 | 2,96 | 12 |

EP 2 073 004 A1

Samples 1 to 4 were measured in their original medium measurements conducted on the Zetasizer Nano ZS device with the standard sample cell DTS1060

| Record | Type | Sample Name | Measurement Date and Time | T °C | ZP mV | Mob μmcm/Vs | Cond mS/cm | Current mA | Voltage V | Quality | Zeta Runs |
|---|---|---|---|---|---|---|---|---|---|---|---|
| 47 | Zeta | sample 4 FI 06.07.07 vesicle_ 3 2 | Friday, July 13 2007 17:46:33 | 25 | -14,8 | -1,158 | 0,654 | 0,337 | 40 | 2,29 | 12 |
| 48 | Zeta | sample 4 FI 06.07.07 vesicle_ 3 3 | Friday, July 13 2007 17:47:52 | 25 | -14,6 | -1,146 | 0,654 | 0,337 | 40 | 1,63 | 12 |
| 49 | Zeta | sample 4 FI 06.07.07 vesicle_ 3 4 | Friday, July 13 2007 17:49:12 | 25 | -14,2 | -1,114 | 0,654 | 0,337 | 40 | 2,42 | 12 |
| 50 | Zeta | sample 4 FI 06.07.07 vesicle_ 3 5 | Friday, July 13 2007 17:50:32 | 25 | -14,7 | -1,149 | 0,654 | 0,337 | 40 | 2,59 | 12 |
| | | **mean value** | | | **-14,16** | **-1,1094** | 0,65027 | 0,33567 | | | |
| | | **STABW N** | | | **0,5817** | **0,0454** | 0,0032 | 0,0017 | | | |

One of the most important conclusions of this study is that ZE-FFE can be employed for the discrimination and characterization of mitochondria exhibiting increasing levels of MOMP. The coexistence of different stages of mitochondrial damage was demonstrated, corresponding to M1-M3. This finding challenges the postulated "all or nothing" nature of PT. Although, mitochondria accumulated in one major stage of PT (M2), ZE-FFE allowed for the detection of an intermediate stage (M1), suggesting that PT-associated MOMP may progress through discrete steps. It will be interesting to see whether further mitochondrial subpopulations emerge, *e.g.* when PT is induced by other triggers.

Subsequent analysis of the separated mitochondria revealed their difference in matrix configuration, outer membrane integrity and Cyt *c* content. Thus, due to the simultaneous detection and separation, a direct analytical profile as well as molecular details of the extent of mitochondrial damage can be obtained. It appears plausible that, due to the severe damage and lack of Cyt *c*, M3 and M2 mitochondria would be unable to supply ATP by oxidative phosphorylation. As a possibility, a majority of M3 and M2 mitochondria would hence be associated with necrosis, while M1 and M0 mitochondria would be associated with apoptosis. Thus, the quantitative ratio of these subpopulations might allow for the prediction of the cell death modality that will ensue.

The comparison of mitochondrial damage inflicted by PT *in vitro* or *in vivo* revealed important qualitative differences. After prolonged incubation and/or addition of higher $Ca^{2+}$-doses, isolated mitochondria manifested drastic mitochondrial damage *in vitro*. This damage progressed that far that IMV vesicles lacking outer membrane were formed. In contrast, mitochondria isolated from I/R-treated livers manifested a less pronounced damage. This finding indicates that PT-induced "large amplitude swelling" did not occur *in vivo* or did not proceed to end stages. It is tempting to speculate that the presence of cytosol (with its considerable ion buffer capacities and colloid osmotic pressure) and/or geometric constraints (induced by the cytoskeleton and the presence of the mitochondrial network) prevent mitochondria from losing large parts of their outer membrane or from being completely spoiled of their envelope as this can occur *in vitro*. Irrespective, of these theoretical considerations, it appears that our approach enables the detection of mitochondrial damage by PT, a direct comparison of the extent of mitochondrial damage upon PT as well as a comparison between mitochondria from PT inflicting *in vivo* situations and *in vitro* experiments.

In conclusion, it can be anticipated that ZE-FFE will provide a welcome addition to the methodological armamentarium of mitochondrial research. ZE-FFE is a novel tool for analyzing and purifying mitochondria that have undergone defined levels of damage.

**References**

Armstrong JS, Yang H, Duan W, and Whiteman M. Cytochrome bc1 regulates the mitochondrial permeability transition by two distinct pathways. Journal of Biological Chemistry. 279 (48), 50420-50428 (2004).

Baines, C.P., Kaiser, R.A., Sheiko, T., Craigen, W.J. & Molkentin, J.D. Voltage-dependent anion channels are dispensable for mitochondrial-dependent cell death. Nat Cell Biol 9, 550-555 (2007).

Beatrice, M.C., Stiers, D.L. & Pfeiffer, D.R. Increased permeability of mitochondria during Ca2+ release induced by t-butyl hydroperoxide or oxalacetate the effect of ruthenium red. J Biol Chem 257, 7161-7171 (1982).

Beatrice, M.C., Palmer, J.W. & Pfeiffer, D.R. The relationship between mitochondrial membrane permeability, membrane potential, and the retention of Ca2+ by mitochondria. J Biol Chem 255, 8663-8671 (1980).

Bernardi, P. et al. The mitochondrial permeability transition from in vitro artifact to disease target. Febs J 273, 2077-2099 (2006).

Bernardi, P., Scorrano, L., Colonna, R., Petronilli, V. & Di Lisa, F. Mitochondria and cell death. Mechanistic and methodological issues. Eur. J. Biochem. 264, 687-701 (1999).

Beutner, G., Rück, A., Riede, B., Brdiczka, D. Complexes between porin, hexokinase, mitochondrial creatine kinase and adenylate translocator display properties of the permeability transition pore. Implication for regulation of permeability transition by the kinases. Biochimica et Biophysica Acta 1368 (1), 7-18 (1998).

Brenner, C. & Grimm, S. The permeability transition pore complex in cancer cell death. Oncogene 25, 4744-4756 (2006).

Brustovetsky N., Brustovetsky T., Jemmerson R. and Dubinsky J.M. Calcium induced cytochrome c release from CNS mitochondria is associated with the permeability transition and rupture of the outer membrane. Journal of Neurochemistry, 80 (2), 207-218 (2002).

Crompton, M., Ellinger, H. & Costi, A. Inhibition by cyclosporin A of a Ca2+-dependent pore in heart mitochondria activated by inorganic phosphate and oxidative stress. Biochem J 255, 357-360 (1988).

Deniaud, A., Sharaf El Dein, O., Maillier, E., Poncet, D., Kroemer, G., Lemaire, C., Brenner, C. Endoplasmic reticulum stress induces calcium-dependent permeability transition, mitochondrial outer membrane permeabilization and apoptosis. Oncogene, August 13, 2007, published online ahead of print.

Fiskum G. Mitochondrial dysfunction in the pathogenesis of acute neuronal cell death. Chapter 16 In Mitochondria in pathogenesis. Lemasters JJ and Nieminen AL, eds. Kluwer Academic/Plenum Publishers. New York. 317 - 331 (2001).

Fleischer, S. Long-term storage of mitochondria to preserve energy-linked functions. Methods Enzymol 55, 28-32 (1979).

Friberg, H. and Wieloch, T. [Mitochondrial permeability transition in acute neurodegeneration.] Biochimie 84 (2-3), 241-250 (2002).

Galluzzi, L. et al. Methods for the assessment of mitochondrial membrane permeabilization in apoptosis. Apoptosis (2007).

Garcia-Ruiz, C., Colell, A., Paris, R. and Fernandez-Checa, J.C. Direct interaction of GD3 ganglioside with mitochondria generates reactive oxygen species followed by mitochondrial permeability transition, cytochrome c release, and caspase activation. FASEB Journal 14 (7), 847-858 (2000).

Green, D.R. & Kroemer, G. The pathophysiology of mitochondrial cell death. Science 305, 626-629 (2004).

Grimm, S. & Brdiczka, D. The permeability transition pore in cell death. Apoptosis 12, 841-855 (2007).

Gunter, T.E., Gunter, K.K., Sheu, S.S. and Gavin, C.E. Mitochondrial calcium transport: physiological and pathological relevance. American journal of Physiology 267(2), C313-C339, 1994.

Halestrap, A.P. Calcium, mitochondria and reperfusion injury: a pore way to die. Biochem Soc Trans 34, 232-237 (2006).

Hannig, K. & Heidrich, H.G. Free-Flow Electrophoresis. (GIT Verlag, Darmstadt; 1990).

Hansen E. und Hannig K. Antigen-specificelectriophoretic cell separation (ASECS) : isolation of human T and B lymphocytesubpopulations by freeflowelectrophoresis after reaction with antibodies, J. Immunol. Methods 11, 197-208 (1982).

Haworth, R.A. and Hunter D.R. The Ca2+-induced membrane transition in mitochondria II. Nature of the Ca2+ trigger site. Archives of Biochemistry and Biophysics, 195 (2), 460-467 (1979).

Heidrich, H.G., Stahn, R. & Hannig, K. The surface charge of rat liver mitochondria and their membranes. Clarification of some controversies concerning mitochondrial structure. J Cell Biol 46, 137-150 (1970).

Hunter, D.R. and Haworth, R.A. The Ca2+-induced membrane transition in mitochondria. Transitional Ca2+ release. Archives of Biochemistry and Biophysics 195 (2), 468-477 (1979).

Hunter, D.R., Haworth, R.A. & Southard, J.H. Relationship between configuration, function, and permeability in calcium-treated mitochondria. J Biol Chem 251, 5069-5077 (1976).

Ichas, F. and Mazat, J.P. From calcium signaling to cell death: two conformations for the mitochondrial permeability transition pore. Switching from low- to high-conductance state. Biochimica et Biophysica Acta, 1366 (1-2), 33-50 (1998).

Kinnally, K.W. & Antonsson, B. A tale of two mitochondrial channels, MAC and PTP, in apoptosis. Apoptosis (2007).

Krivankova, L. & Bocek, P. Continuous free-flow electrophoresis. Electrophoresis 19, 1064-1074 (1998).

Kroemer, G., Galluzzi, L. & Brenner, C. Mitochondrial membrane permeabilization in cell death. Physiol Rev 87, 99-163 (2007).

Kroemer, G., Petit, P., Zamzami, N., Vayssiere, J.L. & Mignotte, B. The biochemistry of programmed cell death. Faseb J 9, 1277-1287 (1995).

Mannella, C.A. The relevance of mitochondrial membrane topology to mitochondrial function. Biochim Biophys Acta 1762, 140-147 (2006).

Masobuchi, Y, Suda, C and Horie, T. Involvement of mitochondrial permeability transition in acetaminophen-induced liver injury in mice. J Hepatol 42, 110-116 (2005).

Nicholls, D.G. and Brand, M.D. The nature of the calcium ion efflux induced in rat liver mitochondria by the oxidation of endogenous nicotinamide nucleotides. Biochemical Journal 188 (1), 113-118, 1980.

Ott, M., Gogvadze, V., Orrenius, S. & Zhivotovsky, B. Mitochondria, oxidative stress and cell death. Apoptosis 12, 913-922 (2007).

Pallotti, F. and Lenaz, G. Isolation and subfractionation of mitochondria from animal cells and tissue culture lines. Methods Cell Biol 80, 3-44 (2007).

Petronilli, V., Cola, C., Massari, S., Colonna, R. & Bernardi, P. Physiological effectors modify voltage sensing by the cyclosporin A-sensitive permeability transition pore of mitochondria. J Biol Chem 268, 21939-21945 (1993).

Rabilloud, T. Two-dimensional gel electrophoresis in proteomics: old, old fashioned, but it still climbs up the mountains. Proteomics 2, 3-10 (2002).

Rodriguez-Enriquez, S., He, L. & Lemasters, J.J. Role of mitochondrial permeability transition pores in mitochondrial autophagy. Int J Biochem Cell Biol 36, 2463-2472 (2004).

Saris, N.E. & Carafoli, E. A historical review of cellular calcium handling, with emphasis on mitochondria. Biochemistry (Mosc) 70, 187-194 (2005).

Saris, N.E. & Eriksson, K.O. Mitochondrial dysfunction in ischaemia-reperfusion. Acta Anaesthesiol Scand Suppl 107, 171-176 (1995).

Schinder, A.F., Olson, E.C., Spitzer, N.C. and Montal, M. Mitochondrial dysfunction is a primary event in glutamate neurotoxicity. Journal of Neuroscience 16, (19), 6125-6133 (1996).

Sorgato, M.C., Keller, B.U. & Stuhmer, W. Patch-clamping of the inner mitochondrial membrane reveals a voltage-dependent ion channel. Nature 330, 498-500 (1987).

Szabo, I. & Zoratti, M. The mitochondrial megachannel is the permeability transition pore. J Bioenerg Biomembr 24, 111-117 (1992).

Towbin, H., Staehelin, T. & Gordon, J. Electrophoretic transfer of proteins from polyacrylamide gels to nitrocellulose sheets: procedure and some applications. Proc Natl Acad Sci U S A 76, 4350-4354 (1979).

Tsujimoto, Y. & Shimizu, S. Role of the mitochondrial membrane permeability transition in cell death. Apoptosis 12, 835-840 (2007).

Van der Heiden, M.G., Chandel, N.S., Williamson, E.K., Schumacker, P.T. & Thompson, C.B. Bcl-xL regulates the membrane potential and volume homeostasis of mitochondria. Cell 91, 627-637 (1997).

Weiss, J.N., Korge, P., Honda, H.M. & Ping, P. Role of the mitochondrial permeability transition in myocardial disease. Circ Res 93, 292-301 (2003).

Zischka, H. et al. Improved proteome analysis of Saccharomyces cerevisiae mitochondria by free-flow electrophoresis. Proteomics 3, 906-916 (2003).

Zischka, H. et al. Differential Analysis of Saccharomyces cerevisiae Mitochondria by Free Flow Electrophoresis. Mol Cell Proteomics 5, 2185-2200 (2006).

**Claims**

1. A method for detecting mitochondrial damage in mammalian tissue or cells comprising:

(a) isolating mitochondria from a sample obtained from a mammal and containing tissue or cells containing mitochondria;
(b1) separating populations of the isolated mitochondria of step (a) by means of their electrophoretic mobility; and
(c1) comparing the populations of the mitochondria obtained in step (b1) with those of a reference sample containing tissue or cells displaying a non-pathological phenotype, wherein a rise in the number of populations, a shift of the population(s) towards the anode or the cathode and/or an increase in the heterogeneity of one or more populations separated in step (b1) as compared to those of the reference sample is indicative of mitochondrial damage; or
(b2) analyzing the average surface charge of the isolated mitochondria of step (a); and
(c2) comparing the average surface charge determined in step (b2) with that of a reference sample containing cells displaying a non-pathological phenotype, wherein a shift of the average surface charge of the mitochondria analyzed in step (b2) as compared to that of the mitochondria of the reference sample is indicative of mitochondrial damage; and
(d) in the case that a shift of the population(s) towards the cathode in step (c1) or a shift of the average surface charge to the positive in step (c2) is detected or, confirming that mitochondrial damage is present.

2. A method of identifying an agent causing mitochondrial damage in mammalian cells comprising:

(a) contacting with a test agent

(i) a non-human animal and obtaining a sample containing tissue or cells to be analysed; or
(ii) a sample obtained from a mammal and containing cells with mitochondria; or
(iii) cultured cells containing mitochondria; or
(iv) isolated mitochondria; and isolating the mitochondria from the sample of (i), (ii) or the cells of (iii);

(b1) separating populations of the isolated mitochondria of step (a) by means of their electrophoretic mobility; and
(c1) comparing the populations of the mitochondria obtained by analysing the electrophoretic mobility in (b1) with those of an untreated sample, wherein a shift of the population(s) towards the anode or the cathode, a rise in the number of mitochondrial populations and/or an increase in the heterogeneity of one or more populations separated in step (b1) as compared to the untreated sample is indicative of mitochondrial damage caused by the agent; or
(b2) analyzing the average surface charge of the isolated mitochondria of step (a); and
(c2) comparing the average surface charge determined in step (b2) with that of an untreated sample, wherein a shift of the average surface charge of the mitochondria analyzed in step (b2) as compared to that of the untreated sample is indicative of mitochondrial damage caused by the agent; and
(d) in the case that a shift of the population(s) towards the cathode or a shift of the average surface charge to the positive is detected, confirming that mitochondrial damage caused by the agent is present

3. A method of identifying an agent capable of regenerating damaged mammalian mitochondria comprising:

(a) contacting with a test agent

(i) a non-human animal containing cells with damaged mitochondria and obtaining a sample containing tissue or cells to be analyzed; or
(ii) a sample obtained from a mammal and containing cells with damaged mitochondria; or
(iii) cultured cells containing damaged mitochondria; or
(iv) isolated damaged mitochondria; and

isolating the mitochondria from the sample of (i), (ii) or the cells of (iii);
(b1) separating populations of the isolated mitochondria of step (a) by means of their electrophoretic mobility; and
(c1) comparing the populations of the mitochondria obtained in (b1) by analysing the electrophoretic mobility with those of a reference sample not contacted with the test agent and containing damaged mitochondria, wherein a shift of the mitochondrial populations towards the cathode, a change in the distribution of the mitochondrial populations and/or a decrease in the heterogeneity of one or more populations separated in step (b1) as compared to the reference sample is indicative of a regeneration of the mitochondria caused by the agent; or
(b2) analyzing the average surface charge of the isolated mitochondria of step (a); and
(c2) comparing the average surface charge determined in step (b2) with that of a reference sample not contacted with the test agent and containing damaged mitochondria, wherein a shift of the average surface charge of the mitochondria analyzed in step (b2) as compared to that of the reference sample is indicative of a regeneration of the mitochondria caused by the agent; and
(d) in the case that a shift of the population(s) towards the anode or a shift of the average surface charge to the negative is detected, confirming that the mitochondria have regenerated.

4. The method of any one of claims 1 to 3, further comprising separating the mitochondria according to their electrophoretic mobility directly prior to the analysis of the average surface charge.

5. The method of any one of claims 1 to 4, wherein the separation according to the electrophoretic mobility is effected by free-flow electrophoresis.

6. The method of any one of claims 1 to 4, wherein the surface charge is analyzed by determining the zeta potential.

7. The method of claim 2, wherein the non-human animal, the sample, the cells or the mitochondria, prior to contacting with the test agent, and the untreated sample of step (c1) or (c2) each display a non-pathological phenotype.

8. The method of any one of claims 2 to 7, wherein the untreated or reference sample of step (c1) or (c2) and the sample, the cells or the mitochondria of step (a) are from the same source.

9. The method of any one of claims 1, 2 and 4 to 8, wherein in the case that a shift of the population(s) towards the anode in step (c1) or a shift of the average surface charge to the negative in step (c2) is detected, the method further comprises confirming that mitochondrial damage is present.

10. The method of any one of claims 3 to 6 and 8, wherein in the case that a shift of the population(s) towards the cathode or a shift of the average surface charge to the positive is detected in step (c1) or (c2), the method further comprises confirming that the mitochondria have regenerated.

11. The method of any one of claims 3, 4 to 6, 8 or 10 comprising alternatively to step (c1) or (c2) or additionally:

(e1) comparing the electrophoretic mobility of the populations of the mitochondria obtained in (b1) with that of populations of a reference sample displaying a non-pathological phenotype, wherein a position, distribution and/or a heterogeneity of the mitochondrial populations similar to one or more of those of the reference sample is indicative of a regeneration of the mitochondria caused by the agent; or
(e2) comparing the average surface charge determined in step (b2) with that of a reference sample displaying a non-pathological phenotype, wherein an average surface charge similar to that of the reference sample is indicative of a regeneration of the mitochondria caused by the agent.

12. The method of any one of claims 1, 2 and 4 to 9, wherein the mitochondrial damage is mitochondrial outer membrane permeabilization (MOMP).

13. The method of any one of claims 1 to 12, wherein the confirmation that mitochondrial damage caused by the agent or a regeneration of the mitochondria caused by the agent is present comprises determining the presence and optionally the quantity of proteins specific for the outer and/or inner mitochondrial membrane in one or more of the populations.

14. The method of claim 1, 2 or 4 to 13, wherein the mitochondrial damage detected results in cell death or regeneration/ survival of the cells.

15. The method of claim 14, wherein cell death is caused by apoptosis or necrosis.

16. The method according to any one of claims 1 to 15, wherein the mitochondrial damage of the mitochondria is indicative of a disease state or condition.

17. The method of claim 16, wherein the disease state or condition is ischemia reperfusion damage, a neurodegenerative disease, a liver disease, cancer, a haematological disease or a viral infection.

18. The method according to any one of claims 1 to 17, further comprising analysing one or more of the obtained populations with one or more of electron microscopy, immunoblotting, immune electrophoresis, immunoprecipitation, cytofluorometric detection, HPLC, enzymatic activity assays, colocalization studies, detection of inner membrane surface metabolites, NMR, light scattering, atomic force microscopy, two-dimensional gel electrophoresis, optionally in combination with mass spectrometric methods for protein identification or liquid chromatographic methods, optionally in combination with mass spectrometry.

19. The method according to any one of claims 2, 4 to 9 and 12 to 18 wherein the test agent is a (cyto)toxic agent.

20. The method according to any one of claims 2 to 19, wherein a library of test agents is screened.

Figure 1A

Figure 1B

Figure 2A

Figure 2B

Figure 2C

Figure 3A

Figure 3B

Figure 3C

Figure 3D

EP 2 073 004 A1

## Figure 4A

38

# Figure 4B

Figure 4C

| | Untreated Control | Ischemia/30 min Reperfusion Anodal Peak | Ischemia/3 h Reperfusion | |
|---|---|---|---|---|
| | | | | VDAC |
| | | | | CYT C |
| | | | | TIM 23 |
| | | | | CYP D |

Surface Charge of mitochondrial subpopulations

Surface charge in mV (negative values)

Control mitochondria FFE purified Preparation 1

Ca2+ treated mitochondria FFE purified Preparation 1

Control mitochondria FFE purified Preparation 2

Ca2+ treated mitochondria FFE purified Preparation 2

Figure 5A

electrophoretic mobility in µm cm/Vs (negative values)

Electrophoretic mobility of mitochondrial subpopulations

Figure 5B

**European Patent Office**

**EUROPEAN SEARCH REPORT**

Application Number

EP 07 02 4623

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2006/185451 A1 (HAN JIN [KR] ET AL) 24 August 2006 (2006-08-24) * page 2, left-hand column, paragraph 26 - paragraph 27 * ----- | 1-20 | INV. G01N33/50 |
| Y | WO 2004/042079 A (UNISEARCH LTD [AU]; HOGG PHILIP JOHN [AU]) 21 May 2004 (2004-05-21) * claims 1-5 * * page 25, line 10 - line 13 * ----- | 1-20 | |
| Y | US 6 204 067 B1 (SIMON SANFORD M [US] ET AL) 20 March 2001 (2001-03-20) * column 13, line 49 - line 55 * ----- | 1-20 | |
| Y | US 2003/110840 A1 (ARRIAGA EDGAR A [US] ET AL) 19 June 2003 (2003-06-19) * the whole document * ----- | 1-20 | |
| Y | WO 00/19200 A (MITOKOR [US]; DYKENS JAMES A [US]; MILLER SCOTT W [US]; GHOSH SOUMITRA) 6 April 2000 (2000-04-06) * page 36, line 19 - line 22 * ----- | 1-20 | TECHNICAL FIELDS SEARCHED (IPC) G01N |

The present search report has been drawn up for all claims

| Place of search | Date of completion of the search | Examiner |
|---|---|---|
| The Hague | 27 February 2008 | Routledge, Brian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 07 02 4623

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2008

| Patent document cited in search report | | Publication date | Patent family member(s) | | Publication date |
|---|---|---|---|---|---|
| US 2006185451 | A1 | 24-08-2006 | KR 20060094239 | A | 29-08-2006 |
| WO 2004042079 | A | 21-05-2004 | CA 2504935 | A1 | 21-05-2004 |
| | | | EP 1563099 | A1 | 17-08-2005 |
| | | | JP 2006518986 | T | 24-08-2006 |
| | | | US 2006166208 | A1 | 27-07-2006 |
| US 6204067 | B1 | 20-03-2001 | NONE | | |
| US 2003110840 | A1 | 19-06-2003 | NONE | | |
| WO 0019200 | A | 06-04-2000 | AU 6162899 | A | 17-04-2000 |
| | | | CA 2345066 | A1 | 06-04-2000 |
| | | | EP 1116027 | A1 | 18-07-2001 |
| | | | JP 2002525630 | T | 13-08-2002 |
| | | | US 2003044776 | A1 | 06-03-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Armstrong JS ; Yang H ; Duan W ; Whiteman M.** Cytochrome bc1 regulates the mitochondrial permeability transition by two distinct pathways. *Journal of Biological Chemistry,* 2004, vol. 279 (48), 50420-50428
- **Baines, C.P. ; Kaiser, R.A. ; Sheiko, T. ; Craigen, W.J. ; Molkentin, J.D.** Voltage-dependent anion channels are dispensable for mitochondrial-dependent cell death. *Nat Cell Biol,* 2007, vol. 9, 550-555
- **Beatrice, M.C. ; Stiers, D.L. ; Pfeiffer, D.R.** Increased permeability of mitochondria during Ca2+ release induced by t-butyl hydroperoxide or oxalacetate the effect of ruthenium red. *J Biol Chem,* 1982, vol. 257, 7161-7171
- **Beatrice, M.C. ; Palmer, J.W. ; Pfeiffer, D.R.** The relationship between mitochondrial membrane permeability, membrane potential, and the retention of Ca2+ by mitochondria. *J Biol Chem,* 1980, vol. 255, 8663-8671
- **Bernardi, P. et al.** The mitochondrial permeability transition from in vitro artifact to disease target. *Febs J,* 2006, vol. 273, 2077-2099
- **Bernardi, P. ; Scorrano, L. ; Colonna, R. ; Petronilli, V. ; Di Lisa, F.** Mitochondria and cell death. Mechanistic and methodological issues. *Eur. J. Biochem.,* 1999, vol. 264, 687-701
- **Beutner, G. ; Rück, A. ; Riede, B. ; Brdiczka, D.** Complexes between porin, hexokinase, mitochondrial creatine kinase and adenylate translocator display properties of the permeability transition pore. Implication for regulation of permeability transition by the kinases. *Biochimica et Biophysica Acta,* 1998, vol. 1368 (1), 7-18
- **Brenner, C. ; Grimm, S.** The permeability transition pore complex in cancer cell death. *Oncogene,* 2006, vol. 25, 4744-4756
- **Brustovetsky N. ; Brustovetsky T. ; Jemmerson R. ; Dubinsky J.M.** Calcium induced cytochrome c release from CNS mitochondria is associated with the permeability transition and rupture of the outer membrane. *Journal of Neurochemistry,* 2002, vol. 80 (2), 207-218
- **Crompton, M. ; Ellinger, H. ; Costi, A.** Inhibition by cyclosporin A of a Ca2+-dependent pore in heart mitochondria activated by inorganic phosphate and oxidative stress. *Biochem J,* 1988, vol. 255, 357-360

- **Deniaud, A. ; Sharaf El Dein, O. ; Maillier, E. ; Poncet, D. ; Kroemer, G. ; Lemaire, C. ; Brenner, C.** Endoplasmic reticulum stress induces calcium-dependent permeability transition, mitochondrial outer membrane permeabilization and apoptosis. *Oncogene,* 13 August 2007
- Mitochondrial dysfunction in the pathogenesis of acute neuronal cell death. **Fiskum G.** Mitochondria in pathogenesis. Kluwer Academic/Plenum Publishers, 2001, 317-331
- **Fleischer, S.** Long-term storage of mitochondria to preserve energy-linked functions. *Methods Enzymol,* 1979, vol. 55, 28-32
- **Friberg, H. ; Wieloch, T.** Mitochondrial permeability transition in acute neurodegeneration. *Biochimie,* 2002, vol. 84 (2-3), 241-250
- **Galluzzi, L. et al.** Methods for the assessment of mitochondrial membrane permeabilization in apoptosis. *Apoptosis,* 2007
- **Garcia-Ruiz, C. ; Colell, A. ; Paris, R. ; Fernandez-Checa, J.C.** Direct interaction of GD3 ganglioside with mitochondria generates reactive oxygen species followed by mitochondrial permeability transition, cytochrome c release, and caspase activation. *FASEB Journal,* 2000, vol. 14 (7), 847-858
- **Green, D.R. ; Kroemer, G.** The pathophysiology of mitochondrial cell death. *Science,* 2004, vol. 305, 626-629
- **Grimm, S. ; Brdiczka, D.** The permeability transition pore in cell death. *Apoptosis,* 2007, vol. 12, 841-855
- **Gunter, T.E. ; Gunter, K.K. ; Sheu, S.S. ; Gavin, C.E.** Mitochondrial calcium transport: physiological and pathological relevance. *American journal of Physiology,* 1994, vol. 267 (2), C313-C339
- **Halestrap, A.P.** Calcium, mitochondria and reperfusion injury: a pore way to die. *Biochem Soc Trans,* 2006, vol. 34, 232-237
- **Hannig, K. ; Heidrich, H.G.** Free-Flow Electrophoresis. GIT Verlag, 1990
- **Hansen E. ; Hannig K.** Antigen-specific electrophoretic cell separation (ASECS) : isolation of human T and B lymphocyte subpopulations by free flow electrophoresis after reaction with antibodies. *J. Immunol. Methods,* 1982, vol. 11, 197-208
- **Haworth, R.A. ; Hunter D.R.** The Ca2+-induced membrane transition in mitochondria II. Nature of the Ca2+ trigger site. *Archives of Biochemistry and Biophysics,* 1979, vol. 195 (2), 460-467

- **Heidrich, H.G. ; Stahn, R. ; Hannig, K.** The surface charge of rat liver mitochondria and their membranes. Clarification of some controversies concerning mitochondrial structure. *J Cell Biol,* 1970, vol. 46, 137-150
- **Hunter, D.R. ; Haworth, R.A.** The Ca2+-induced membrane transition in mitochondria. Transitional Ca2+ release. *Archives of Biochemistry and Biophysics,* 1979, vol. 195 (2), 468-477
- **Hunter, D.R. ; Haworth, R.A. ; Southard, J.H.** Relationship between configuration, function, and permeability in calcium-treated mitochondria. *J Biol Chem,* 1976, vol. 251, 5069-5077
- **Ichas, F. ; Mazat, J.P.** From calcium signaling to cell death: two conformations for the mitochondrial permeability transition pore. Switching from low- to high-conductance state. *Biochimica et Biophysica Acta,* 1998, vol. 1366 (1-2), 33-50
- **Kinnally, K.W. ; Antonsson, B.** A tale of two mitochondrial channels, MAC and PTP, in apoptosis. *Apoptosis,* 2007
- **Krivankova, L. ; Bocek, P.** Continuous free-flow electrophoresis. *Electrophoresis,* 1998, vol. 19, 1064-1074
- **Kroemer, G. ; Galluzzi, L. ; Brenner, C.** Mitochondrial membrane permeabilization in cell death. *Physiol Rev,* 2007, vol. 87, 99-163
- **Kroemer, G. ; Petit, P. ; Zamzami, N. ; Vayssiere, J.L. ; Mignotte, B.** The biochemistry of programmed cell death. *Faseb J,* 1995, vol. 9, 1277-1287
- **Mannella, C.A.** The relevance of mitochondrial membrane topology to mitochondrial function. *Biochim Biophys Acta,* 2006, vol. 1762, 140-147
- **Masobuchi, Y ; Suda, C ; Horie, T.** Involvement of mitochondrial permeability transition in acetaminophen-induced liver injury in mice. *J Hepatol,* 2005, vol. 42, 110-116
- **Nicholls, D.G. ; Brand, M.D.** The nature of the calcium ion efflux induced in rat liver mitochondria by the oxidation of endogenous nicotinamide nucleotides. *Biochemical Journal,* 1980, vol. 188 (1), 113-118
- **Ott, M. ; Gogvadze, V. ; Orrenius, S. ; Zhivotovsky, B.** Mitochondria, oxidative stress and cell death. *Apoptosis,* 2007, vol. 12, 913-922
- **Pallotti, F. ; Lenaz, G.** Isolation and subfractionation of mitochondria from animal cells and tissue culture lines. *Methods Cell Biol,* 2007, vol. 80, 3-44
- **Petronilli, V. ; Cola, C. ; Massari, S. ; Colonna, R. ; Bernardi, P.** Physiological effectors modify voltage sensing by the cyclosporin A-sensitive permeability transition pore of mitochondria. *J Biol Chem,* 1993, vol. 268, 21939-21945
- **Rabilloud, T.** Two-dimensional gel electrophoresis in proteomics: old, old fashioned, but it still climbs up the mountains. *Proteomics,* 2002, vol. 2, 3-10
- **Rodriguez-Enriquez, S. ; He, L. ; Lemasters, J.J.** Role of mitochondrial permeability transition pores in mitochondrial autophagy. *Int J Biochem Cell Biol,* 2004, vol. 36, 2463-2472
- **Saris, N.E. ; Carafoli, E.** A historical review of cellular calcium handling, with emphasis on mitochondria. *Biochemistry (Mosc),* 2005, vol. 70, 187-194
- **Saris, N.E. ; Eriksson, K.O.** Mitochondrial dysfunction in ischaemia-reperfusion. *Acta Anaesthesiol Scand,* 1995, 171-176
- **Schinder, A.F. ; Olson, E.C. ; Spitzer, N.C. ; Montal, M.** Mitochondrial dysfunction is a primary event in glutamate neurotoxicity. *Journal of Neuroscience,* 1996, vol. 16 (19), 6125-6133
- **Sorgato, M.C. ; Keller, B.U. ; Stuhmer, W.** Patch-clamping of the inner mitochondrial membrane reveals a voltage-dependent ion channel. *Nature,* 1987, vol. 330, 498-500
- **Szabo, I. ; Zoratti, M.** The mitochondrial megachannel is the permeability transition pore. *J Bioenerg Biomembr,* 1992, vol. 24, 111-117
- **Towbin, H. ; Staehelin, T. ; Gordon, J.** Electrophoretic transfer of proteins from polyacrylamide gels to nitrocellulose sheets: procedure and some applications. *Proc Natl Acad Sci U S A,* 1979, vol. 76, 4350-4354
- **Tsujimoto, Y. ; Shimizu, S.** Role of the mitochondrial membrane permeability transition in cell death. *Apoptosis,* 2007, vol. 12, 835-840
- **Van der Heiden, M.G. ; Chandel, N.S. ; Williamson, E.K. ; Schumacker, P.T. ; Thompson, C.B.** Bcl-xL regulates the membrane potential and volume homeostasis of mitochondria. *Cell,* 1997, vol. 91, 627-637
- **Weiss, J.N. ; Korge, P. ; Honda, H.M. ; Ping, P.** Role of the mitochondrial permeability transition in myocardial disease. *Circ Res,* 2003, vol. 93, 292-301
- **Zischka, H. et al.** Improved proteome analysis of Saccharomyces cerevisiae mitochondria by free-flow electrophoresis. *Proteomics,* 2003, vol. 3, 906-916
- **Zischka, H. et al.** Differential Analysis of Saccharomyces cerevisiae Mitochondria by Free Flow Electrophoresis. *Mol Cell Proteomics,* 2006, vol. 5, 2185-2200